# EUROPEAN PATENT SPECIFICATION

(11) **EP 3 194 627 B1**
(45) Date of publication and mention of the grant of the patent: **16.08.2023**
(21) Application number: 15842246.9
(22) Date of filing: 15.09.2015
(51) Int. Cl.: G16B 20/20, G16B 30/10, G16B 20/10

(54) **METHODS AND SYSTEMS FOR ANALYZING NUCLEIC ACID SEQUENCING DATA**
VERFAHREN UND SYSTEME ZUR ANALYSE VON NUKLEINSÄURESEQUENZIERUNGSDATEN
PROCÉDÉS ET SYSTÈMES POUR ANALYSER DES DONNÉES DE SÉQUENÇAGE D'ACIDE NUCLÉIQUE

(30) Priority: 18.09.2014 US 201462052189 P
(43) Date of publication of application: 26.07.2017
(73) Proprietor: Illumina, Inc., San Diego, CA 92122 (US)
(72) Inventor: BRUAND, Jocelyne, San Diego, CA 92122 (US); SCHLESINGER, Johann, Felix, San Diego, CA 92122 (US)
(74) Representative: Murgitroyd & Company
(86) International application number: PCT/US2015/050129
(87) International publication number: WO 2016/044233

(56) References cited:
- EP-A2- 2 287 307
- WO-A1-2013/055817
- WO-A1-2013/148970
- WO-A1-2014/074611
- WO-A1-2014/142831
- WO-A2-2005/086770
- WO-A2-2013/052557
- WO-A2-2013/076586
- US-A1- 2004 215 401
- US-A1- 2012 053 845
- US-A1- 2012 173 159
- US-A1- 2012 270 212
- US-A1- 2014 065 613
- US-A1- 2014 163 900
- US-A1- 2014 163 900
- US-A1- 2014 206 547
- US-B1- 6 274 317
- US-B1- 6 807 490
- US-B2- 8 090 543
- US-B2- 8 825 412

## Description

### BACKGROUND

Various genetic loci have been identified that are useful in differentiating individuals within a species population (e.g., humans) or providing other useful information about the population or individuals within the population. For example, a genetic locus may have a number of variant forms, called alleles, and each individual in a population may have one or more of the alleles for a particular locus. An allele of a locus may differ from other alleles of the same locus in length (i.e., total number of nucleotides) and/or in the sequence of the nucleotides. Various genetic applications exist that analyze the alleles of the genetic loci. These genetic applications include paternity testing, human identification (e.g., forensic analysis), chimera monitoring (e.g., tissue transplantation monitoring), and other genetic applications in plant and animal research. Many genetic applications analyze loci that include short tandem repeats (STRs) and/or single nucleotide polymorphisms (SNPs). STRs are repetitive regions of DNA that include repeat motifs. The repeat motifs, may be, for example, two to six nucleotides in length, although repeat motifs of other sizes exist.

Prior art document US2014163900 discloses methods of analyzing short tandem repeats from high-throughput sequencing. The methods include steps of determining the presence of "stutter artefacts" by probabilities based on repeat length.

Document EP2287307 discloses methods of analyzing intermediate tandem repeat sequences, and addresses the problem of "stutter artefacts" which occur through addition or deletion of one or more repeats during sequencing.

Although STR and/or SNP analysis has improved in recent years, challenges still exist. For instance, analysis of STRs has generally not included analysis of the actual sequence of nucleotides. STRs are typically analyzed using capillary electrophoresis (CE) systems. CE systems only determine a length of an allele, however, and do not identify the sequence of the allele. Thus, it is possible that CE data would indicate that an individual is homozygous for a particular allele when, in fact, the individual has two different alleles that have the same length but different sequences.

Quality control challenges may also exist for systems that analyze nucleic acid sequences. For instance, some assays include preparing a biological sample, amplifying STR alleles of the biological sample, and then sequencing the resulting amplicons. After preparation and amplification of a sample, it may be possible that one or more of the amplicons were developed through primer dimer and/or include nucleic acids from more than one source (e.g., chimeras) rendering the corresponding data unreliable. If the unwanted data is not identified and filtered out, it is may be more difficult to, for example, provide an accurate genetic profile of the source or identify that there are multiple sources. If the unwanted data is identified, the data is typically filtered out and discarded, but without further analysis. Similarly, errors that occur during sequencing may also render analysis more difficult and such data is typically discarded. Lastly, it can also be challenging to reliably determine the gender of an individual from an unknown source.

Accordingly, there is a need for improved methods and systems for analyzing sequencing data.

### BRIEF SUMMARY

Amethod is provided that comprises: receiving sequencing data including a plurality of sample reads that have corresponding sequences of nucleotides;
assigning the sample reads to designated loci based on the sequence of the nucleotides, wherein the sample reads that are assigned to a corresponding designated locus are assigned reads of the corresponding designated locus;
analyzing the assigned reads for each designated locus to identify corresponding regions-of-interest (ROIs) within the assigned reads, each of the ROIs having one or more series of repeat motifs in which each repeat motif of a corresponding series includes an identical set of the nucleotides;
sorting, for designated loci having multiple assigned reads, the assigned reads based on the sequences of the ROIs such that the ROIs with different sequences are assigned as different potential alleles, each potential allele having a sequence that is different from the sequences of other potential alleles within the designated locus;
analyzing, for designated loci having multiple potential alleles, the sequences of the potential alleles to determine whether a first allele of the potential alleles is suspected stutter product of a second allele of the potential alleles, the first allele being the suspected stutter product of the second allele if *k* repeat motifs within the corresponding sequences have been added or dropped between the first and second alleles, wherein k is a whole number, and wherein the total number of sample reads of the first allele is less than the total number of sample reads of the second allele; and
counting, for each designated locus having multiple potential alleles, a total number of the sample reads called for the first allele and for the second allele;
wherein, if the first allele is the suspected stutter product of the second allele:
   (i) the first allele is the stutter product of the second allele if the total number of sample reads called for the first allele is within a predetermined percentile range of the total number of sample reads called for the second allele and no other mismatches exist between the sequences of the ROIs of the first and second alleles;
   (ii) the first allele is from another contributor if the total number of sample reads called for the first allele is greater than the predetermined percentile range of the total number of sample reads called for the second allele; and
   (iii) the first allele is designated as noise if the total number of sample reads called for the first allele is less than the predetermined percentile range of the total number of sample reads called for the second allele; and
wherein each of the steps in the method are performed using a suitably programmed computer. Optionally, k is equal to 1 or 2.

Also described is a method that includes receiving sequencing data having a plurality of sample reads of amplicons that correspond to a set of genetic loci. The sample reads include read pairs in which each read pair of a corresponding amplicon includes first and second reads of the corresponding amplicon. Each of the first and second reads has a respective read sequence. The method also includes identifying potential genetic loci for the first reads based on analysis of the read sequences of the first reads. The potential genetic loci are from the set of genetic loci. The method also includes determining, for each of the first reads having at least one potential locus, whether the first read aligns with a reference sequence of each of the potential genetic loci. If the first read aligns with a reference sequence of only one genetic locus, the method includes determining that the first read includes a potential allele of the one genetic locus. If the first read aligns with more than one reference sequence, the method includes determining that the first read includes a potential allele of the genetic locus having the reference sequence that best aligns with the first read. If the first read does not align with a reference sequence, the method includes designating the first read as an unaligned read and analyzing the unaligned read to identify a genetic locus from the potential genetic loci that best fits with the unaligned read. The method also includes generating a genetic profile that includes called genotypes for at least a plurality of the genetic loci, wherein the called genotypes are based on the potential alleles of the corresponding genetic loci. The genetic profile also includes one or more notifications for genetic loci having unaligned reads.

Also described is a method that includes receiving sequencing data having a plurality of sample reads of amplicons that correspond to a set of genetic loci. The sample reads include read pairs in which each read pair of a corresponding amplicon includes first and second reads of the corresponding amplicon. Each of the first and second reads has a respective read sequence. The method also includes identifying potential genetic loci for the first reads based on analysis of the read sequences of the first reads. The potential genetic loci are from the set of genetic loci. The method also includes determining, for each of the first reads having at least one potential locus, whether the first read aligns with a reference sequence of each of the potential genetic loci. The method also includes designating the first reads that do not align with a reference sequence as unaligned reads. The method also includes analyzing the unaligned reads to identify a genetic locus from the potential genetic loci that best fits with the unaligned read. The method also includes analyzing the unaligned reads to determine whether a potential allele dropout exists for the best-fit genetic locus.

Also described is a method that includes receiving a read distribution for each genetic locus of a plurality of genetic loci. The read distribution includes a plurality of potential alleles, wherein each potential allele has an allele sequence and a read count. The read count represents a number of sample reads from sequencing data that were determined to include the potential allele. The method may also include identifying, for each genetic locus of the plurality of genetic loci, one of the potential alleles of the read distribution that has a maximum read count. The method may also include determining, for each genetic locus of the plurality of genetic loci, whether the maximum read count exceeds an interpretation threshold. If the maximum read exceeds the interpretation threshold, the method includes analyzing the potential allele(s) of the corresponding genetic locus to call a genotype for the genetic locus. If the maximum read is less than the interpretation threshold, the method includes generating an alert that the genetic locus has low coverage. The method also includes generating a genetic profile that has the genotypes for each of the genetic loci for which a genotype was called and the alert(s) for genetic loci that have low coverage.

Also described is a method that includes: (a) receiving a read distribution for a genetic locus. The read distribution includes a plurality of potential alleles, wherein each potential allele has an allele sequence and a count score. The count score is based on a number of sample reads from sequencing data that were determined to include the potential allele. The method also includes: (b) determining whether the genetic locus has low coverage based on the count score of one more of the potential alleles. If the genetic locus has low coverage, the method includes generating a notice that the genetic locus has low coverage. If the genetic locus does not have low coverage, the method includes analyzing the count scores of the potential alleles to determine a genotype of the genetic locus. The method also includes: (d) generating a genetic profile that includes the genotype for the genetic locus or the alert that the genetic locus has low coverage.

Also described is a method that includes receiving a read distribution for a genetic locus. The read distribution includes a plurality of potential alleles, wherein each potential allele has an allele sequence and a read count. The read count represents a number of sample reads from sequencing data that were assigned to the genetic locus. The method may also include determining a count score for each of the potential alleles. The count score may be based on the read count of the potential allele. The method may also include determining whether the count scores of the potential alleles pass an analytical threshold. If the count score of a corresponding potential allele does not pass the analytical threshold, the method includes discarding the corresponding potential allele. If the count score of a corresponding potential allele passes the analytical threshold, the method includes designating the potential allele as a designated allele of the genetic locus.

Also described is a method that includes receiving a read distribution for a genetic locus. The read distribution includes a plurality of potential alleles, wherein each potential allele has an allele sequence and a read count. The read count represents a number of sample reads from sequencing data that were assigned to the genetic locus. The method also includes determining whether the read counts exceed an analytical threshold. If the read count of a corresponding potential allele is less than the analytical threshold, the method includes designating the corresponding potential allele as a noise allele. If the read count of a corresponding potential allele passes the analytical threshold, the method includes designating the potential allele as an allele of the genetic locus. The method also includes determining whether a sum of the read counts of the noise alleles exceeds a noise threshold. If the sum exceeds the noise threshold, the method includes generating an alert that the genetic locus has excessive noise.

Also described is a method that includes receiving locus data for each genetic locus of a plurality of genetic loci. The locus data includes one or more designated alleles for the corresponding genetic locus. Each designated allele is based on read counts obtained from sequencing data. The method also includes determining, for each genetic locus of the plurality of genetic loci, whether a number of designated alleles for the corresponding genetic locus is greater than a predetermined maximum number of allowable alleles for the corresponding genetic locus. The method may include generating an allele-number alert if the number of designated alleles exceeds the predetermined maximum number of allowable alleles. The method also includes determining, for each genetic locus of the plurality of genetic loci, whether an allele proportion of the designated alleles is insufficient. The allele proportion may be based on read counts of the designated alleles. The method may also include generating an allele-proportion alert if the allele proportion is unbalanced. The method may also include determining that the sample includes a mixture of a plurality of sources based on a number of allele-number alert(s) and allele-proportion alerts(s) for the set of genetic loci.

Also described is a method that includes receiving locus data for a plurality of Y-loci. The locus data include designated alleles for the Y-loci. Each designated allele is based on read counts obtained from sequencing data. The method also includes comparing a number of designated alleles for each Y-locus to an expected number of alleles for the Y-loci. The method also includes generating a prediction that the sample is male or female based on results from the comparing operation. Optionally, the genetic loci include short tandem repeat (STR) loci and single nucleotide polymorphism (SNP) loci.

### BRIEF DESCRIPTION OF THE DRAWINGS

Figure 1 is a flowchart illustrating a method in accordance with one embodiment.
Figure 2 is a flowchart illustrating a method of designating different types of sample reads for different analyses.
Figure 3 is a schematic diagram illustrating a portion of the method of Figure 2.
Figure 4 is a schematic diagram illustrating how a region of interest (ROI) may be identified in accordance with an embodiment.
Figure 5 is a schematic showing various mis-alignment errors that can occur if the flanking region immediately adjacent to the STR is used to seed the alignment.
Figure 6A is a set of graphs showing actual STR calling compared to theoretical results based on sample input from a mixture of samples.
Figure 6B is another set of graphs showing actual STR calling compared to theoretical results based on sample input from a mixture of samples.
Figure 6C is another set of graphs showing actual STR calling compared to theoretical results based on sample input from a mixture of samples.
Figure 6D is another set of graphs showing actual STR calling compared to theoretical results based on sample input from a mixture of samples.
Figure 7 is a table showing concordance for allele calls for known loci of five control DNA samples.
Figure 8 is a flowchart illustrating a method of identifying stutter product within sample reads in accordance with an embodiment.
Figure 9 includes a table illustrating read counts for potential alleles of the D1S1656 locus.
Figure 10 includes a graph that is based on the data found in the table of Figure 9.
Figure 11 is a flowchart illustrating a method of analyzing sample reads to determine a genotype of one or more genetic loci.
Figure 12 is a flowchart illustrating a method of generating a sample report that includes a plurality of genotype calls.
Figure 13 is a flowchart illustrating a method of detecting whether a sample includes a mixture of sources.
Figure 14 is a flowchart illustrating a method of determining a gender of a sample.
Figure 15 illustrates a system formed in accordance with an embodiment that may be used to carry out various methods set forth herein.
Figure 16A illustrates a portion of a sample report in accordance with one or more embodiments.
Figure 16B illustrates another portion of the sample report in accordance with one or more embodiments.
Figure 17A illustrates a portion of a sample report in accordance with one or more embodiments.
Figure 17B illustrates another portion of the sample report.
Figure 17C illustrates another portion of the sample report.
Figure 17D illustrates another portion of the sample report.
Figure 17E illustrates another portion of the sample report.
Figure 17F illustrates another portion of the sample report.

### DETAILED DESCRIPTION

The present application includes subject matter that is similar to the subject matter described in International Application No. PCT/US2013/030867 (Publication No. WO 2014/142831), filed on March 15, 2013 and entitled "METHODS AND SYSTEMS FOR ALIGNING REPETITIVE DNA ELEMENTS,".

Embodiments set forth herein may be applicable to analyzing nucleic acid sequences to identify sequence variations. Embodiments may be used to analyze potential alleles of a genetic locus and determine a genotype of the genetic locus or, in other words, provide a genotype call for the locus. In some cases, the method and systems set forth herein may generate sample reports or genetic profiles that include a plurality of such genotype calls. Embodiments may also be applicable to monitoring a quality of an assay that includes sequencing and/or analysis of nucleic acid sequences, such as those that include sequence variations. The sequence variations may include single nucleotide polymorphisms (SNPs) or polymorphic, repetitive elements, such as short tandem repeats (STRs). The sequence variations may be located within designated genetic loci, such as those found within the Combined DNA Index System (CODIS) database or otherwise used in genetic analysis. For example, the sequence variations may include STRs selected from the CODIS autosomal STR loci, CODIS Y-STR loci, EU autosomal STR loci, EU Y-STR loci, and the like. The CODIS is a set of core STR loci identified by the FBI laboratory and includes 13 loci: CSF1PO, FGA, TH01, TPOX, VWA, D3S1358, D5S818, D7S820, D8S1179, D13S317, D16S539, D18S51 and D21S11. Additional STRs of interest may include PENTA D and PENTA E, however, other STRs may be analyzed by embodiments set forth herein. The SNPs may be within known databases, such as the National Center for Biotechnology Information (NCBI) dbSNP database. STRs and SNPs may be identified in future research as well.

As used herein, the term "sequence" includes or represents a strand of nucleotides coupled to each other. The nucleotides may be based on DNA or RNA. It should be understood that one sequence may include multiple sub-sequences. For example, a single sample read (e.g., of a PCR amplicon) may have a sequence that has 350 nucleotides. The sample read may include multiple sub-sequences within these 350 nucleotides. For instance, the sample read may include first and second flanking sub-sequences having, for example, 20-50 nucleotides. The first and second flanking sub-sequences may be located on either side of a repetitive segment having a corresponding sub-sequence (e.g., 40-100 nucleotides). Each of the flanking sub-sequences may include (or include portions of) a primer sub-sequence (e.g. 10-30 nucleotides). For ease of reading, the term "sub-sequence" will be referred to as "sequence," but it is understood that two sequences are not necessarily separate from each other on a common strand. To differentiate the various sequences described herein, the sequences may be given different labels (e.g., target sequence, primer sequence, flanking sequence, reference sequence, and the like). Other terms, such as "allele," may be given different labels to differentiate between like objects.

As used herein, the term "region-of-interest" or "ROI" includes a repetitive segment of the sample read that includes one or more series of repeat motifs. The series of repeat motifs may be an STR. In some embodiments, the ROI is only the repetitive segment (e.g., the STR). In other embodiments, however, the ROI may include sub-sequences of flanking regions. For example, the ROI may include the repetitive segment, about 1-5 nucleotides of the first flanking region that extends from one end of the repetitive segment, and about 1-5 nucleotides of the second flanking region that is extends from the opposite end of the repetitive segment.

It should be understood that a repetitive segment is not required to have the same motifs throughout. A repetitive segment may include a series of X-motifs, then a series of Y-motifs, then a series of Z-motifs (or another series of X-motifs), etc. The repetitive segment of [TAGA]11[TAGG]1[TG]5 is one specific example of the above. It should also be understood that a repetitive segment is not required to have repeating motifs throughout. As shown in the above example, a repetitive segment may include repeat motifs that are interrupted by a non-repeating motif. The [TAGG] in the above example is one such non-repeating motif.

As used herein, the term "threshold" indicates a point at which a course of analysis may be changed and/or a point at which an action may be triggered. A threshold is not required to be a predetermined number. Instead, the threshold may be, for instance, a function that is based on a plurality of factors. In other words, the threshold may be adaptive to the circumstances. As an example, when determining whether a plurality of sample reads constitutes noise that should be discarded or data that should be further analyzed, the threshold may be either a set number (e.g., 10 sample reads) or a function that is based on different factors, such as the number of total reads for the corresponding genetic locus and historical knowledge of the genetic locus. Moreover, a threshold may indicate an upper limit, a lower limit, or a range between limits. The action that may be triggered may include, for example, notifying an end user that the sample is suspected of including stutter product, that the sample contains a mixture of sources, that the assay has particular problem areas, that the sample is of poor quality, etc.

In some embodiments, a metric or score that is based on sequencing data may be compared to the threshold. As used herein, the terms "metric" or "score" may include values or results that were determined from the sequencing data or may include functions that are based on the values or results that were determined from the sequencing data. Like a threshold, the metric or score may be adaptive to the circumstances. For instance, the metric or score may be a normalized value.

As an example of a score or metric, one or more embodiments may use count scores when analyzing the data. A count score may be based on number of sample reads. The sample reads may have undergone one or more filtering stages such that the sample reads have at least one common characteristic or quality. For example, each of the sample reads that are used to determine a count score may have been aligned with a reference sequence or may be assigned as a potential allele. The number of sample reads having a common characteristic may be counted to determine a read count. Count scores may be based on the read count. In some embodiments, the count score may be a value that is equal to the read count. In other embodiments, the count score may be based on the read count and other information. For example, a count score may be based on the read count for a particular allele of a genetic locus and a total number of reads for the genetic locus. In some embodiments, the count score may be based on the read count and previously-obtained data for the genetic locus. In some embodiments, the count scores may be normalized scores between predetermined values. The count score may also be a function of read counts from other loci of a sample or a function of read counts from other samples that were concurrently run with the sample-of-interest. For instance, the count score may be a function of the read count of a particular allele and the read counts of other loci in the sample and/or the read counts from other samples. As one example, the read counts from other loci and/or the read counts from other samples may be used to normalize the count score for the particular allele.

Read counts are typically determined from the sequencing data. The read count may be, for example, a number of sample reads that have been determined to have the same ROI that includes the ROI. The read count (e.g., 350 sample reads) may be used to calculate a stutter metric that is then compared to a designated threshold. For example, the stutter metric may be determined by multiplying the read count by a designated factor that is based on historical knowledge, knowledge of the sample, knowledge of the locus, etc. The stutter metric may be a normalized value of the read count.

The above and the following detailed description of various embodiments will be better understood when read in conjunction with the appended drawings. To the extent that the figures illustrate diagrams of the functional blocks of the various embodiments, the functional blocks are not necessarily indicative of the division between hardware circuitry. Thus, for example, one or more of the functional blocks (e.g., modules, processors, or memories) may be implemented in a single piece of hardware (e.g., a general purpose signal processor or a block of random access memory, hard disk, or the like) or multiple pieces of hardware. Similarly, the programs may be stand alone programs, may be incorporated as subroutines in an operating system, may be functions in an installed software package, and the like. It should be understood that the various embodiments are not limited to the arrangements and instrumentality shown in the drawings.

The present application describes various methods and systems for carrying out the methods. At least some of the methods are illustrated in the figures as a plurality of steps. However, it should be understood that embodiments are not limited to the steps illustrated in the figures. Steps may be omitted, steps may be modified, and/or other steps may be added. By way of example, although some embodiments described herein may include preparing and sequencing a sample to obtain sequencing data, other embodiments may include receiving the sequencing data directly, without preparing the sample and/or sequencing the sample. Moreover, steps described herein may be combined, steps may be performed simultaneously, steps may be performed concurrently, steps may be split into multiple sub-steps, steps may be performed in a different order, or steps (or a series of steps) may be re-performed in an iterative fashion. In addition, although different methods are set forth herein, it should be understood that the different methods (or steps of the different methods) may be combined in other embodiments.

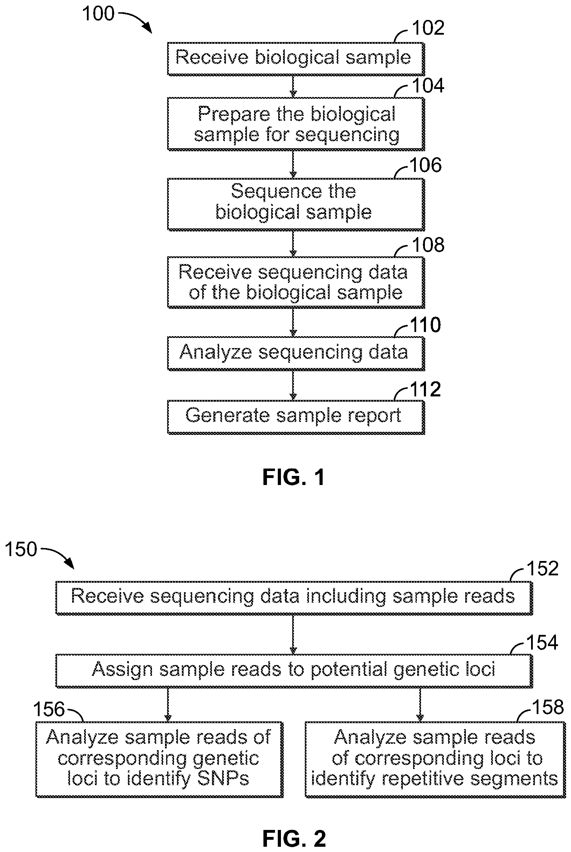

Figure 1 illustrates a method 100 in accordance with one embodiment. The method 100 includes receiving, at 102, a biological sample that includes or is suspected of including nucleic acids, such as DNA. The biological sample may be from a known or unknown source, such as an animal (e.g., human), plant, bacteria, or fungus. The biological sample may be taken directly from the source. For instance, blood or saliva may be taken directly from an individual. Alternatively, the sample may not be obtained directly from the source. For example, the biological sample may be obtained from a crime scene, remains from excavations, or other areas that are being investigated (e.g., a historical site). As used herein, the term "biological sample" includes the possibility that the biological sample has multiple biological samples from different sources. For example, a biological sample obtained through a crime scene may include a mixture of DNA from different individuals.

The method 100 may also include preparing, at 104, the sample for sequencing. The preparation 104 may include removing extraneous material and/or isolating certain material (e.g., DNA). The biological sample may be prepared to include features that are required for a particular assay. For example, the biological sample may be prepared for sequencing-by-synthesis (SBS). In certain embodiments, the preparing may include amplification of certain regions of a genome. For instance, the preparing, at 104, may include amplifying predetermined genetic loci that are known to include STRs and/or SNPs. The genetic loci may be amplified using predetermined primer sequences.

At 106, the sample may be sequenced. The sequencing may be performed through a variety of known sequencing protocols. In particular embodiments, the sequencing includes SBS. In SBS, a plurality of fluorescently-labeled nucleotides are used to sequence a plurality of clusters of amplified DNA (possibly millions of clusters) present on the surface of an optical substrate (e.g., a surface that at least partially defines a channel in a flow cell). The flow cells may contain nucleic acid samples for sequencing where the flow cells are placed within the appropriate flow cell holders. The samples for sequencing can take the form of single nucleic acid molecules that are separated from each other so as to be individually resolvable, amplified populations of nucleic acid molecules in the form of clusters or other features, or beads that are attached to one or more molecules of nucleic acid.

The nucleic acids can be prepared such that they comprise a known oligonucleotide primer, which may be referred to as a primer sequence, that is adjacent to an unknown target sequence. To initiate the first SBS sequencing cycle, one or more differently labeled nucleotides, and DNA polymerase, etc., can be flowed into/through the flow cell by a fluid flow subsystem (not shown). Either a single type of nucleotide can be added at a time, or the nucleotides used in the sequencing procedure can be specially designed to possess a reversible termination property, thus allowing each cycle of the sequencing reaction to occur simultaneously in the presence of several types of labeled nucleotides (e.g. A, C, T, G). The nucleotides can include detectable label moieties such as fluorophores. Where the four nucleotides are mixed together, the polymerase is able to select the correct base to incorporate and each sequence is extended by a single base. Nonincorporated nucleotides can be washed away by flowing a wash solution through the flow cell. One or more lasers may excite the nucleic acids and induce fluorescence. The fluorescence emitted from the nucleic acids is based upon the fluorophores of the incorporated base, and different fluorophores may emit different wavelengths of emission light. A deblocking reagent can be added to the flow cell to remove reversible terminator groups from the DNA strands that were extended and detected. The deblocking reagent can then be washed away by flowing a wash solution through the flow cell. The flow cell is then ready for a further cycle of sequencing starting with introduction of a labeled nucleotide as set forth above. The fluidic and detection steps can be repeated several times to complete a sequencing run. Exemplary sequencing methods are described, for example, in Bentley et al., Nature 456:53-59 (2008), International Publication No. WO 04/018497; U.S. Pat. No. 7,057,026; International Publication No. WO 91/06678; International Publication No. WO 07/123744; U.S. Pat. No. 7,329,492; U.S. Pat. No. 7,211,414; U.S. Pat. No. 7,315,019; U.S. Pat. No. 7,405,281, and U.S. Publication No. 2008/0108082.

In some embodiments, nucleic acids can be attached to a surface and amplified prior to or during sequencing. For example, amplification can be carried out using bridge amplification to form nucleic acid clusters on a surface. Useful bridge amplification methods are described, for example, in U.S. Pat. No. 5,641,658; U.S. Patent Publ. No. 2002/0055100; U.S. Pat. No. 7,115,400; U.S. Patent Publ. No. 2004/0096853; U.S. Patent Publ. No. 2004/0002090; U.S. Patent Publ. No. 2007/0128624; and U.S. Patent Publ. No. 2008/0009420. Another useful method for amplifying nucleic acids on a surface is rolling circle amplification (RCA), for example, as described in Lizardi et al., Nat. Genet. 19:225-232 (1998) and U.S. Patent Publ. No. 2007/0099208 A1.

A particularly useful SBS protocol exploits modified nucleotides having removable 3' blocks, for example, as described in International Publication No. WO 04/018497, U.S. Patent Publication No. 2007/0166705A1, and U.S. Pat. No. 7,057,026. For example, repeated cycles of SBS reagents can be delivered to a flow cell having target nucleic acids attached thereto, for example, as a result of the bridge amplification protocol. The nucleic acid clusters can be converted to single stranded form using a linearization solution. The linearization solution can contain, for example, a restriction endonuclease capable of cleaving one strand of each cluster. Other methods of cleavage can be used as an alternative to restriction enzymes or nicking enzymes, including inter alia chemical cleavage (e.g., cleavage of a diol linkage with periodate), cleavage of abasic sites by cleavage with endonuclease (for example `USER', as supplied by NEB, Ipswich, MA, USA, part number M5505S), by exposure to heat or alkali, cleavage of ribonucleotides incorporated into amplification products otherwise comprised of deoxyribonucleotides, photochemical cleavage or cleavage of a peptide linker. After the linearization step a sequencing primer can be delivered to the flow cell under conditions for hybridization of the sequencing primer to the target nucleic acids that are to be sequenced.

The flow cell can then be contacted with an SBS extension reagent having modified nucleotides with removable 3' blocks and fluorescent labels under conditions to extend a primer hybridized to each target nucleic acid by a single nucleotide addition. Only a single nucleotide is added to each primer because once the modified nucleotide has been incorporated into the growing polynucleotide chain complementary to the region of the template being sequenced there is no free 3'-OH group available to direct further sequence extension and therefore the polymerase cannot add further nucleotides. The SBS extension reagent can be removed and replaced with scan reagent containing components that protect the sample under excitation with radiation. Exemplary components for scan reagent are described in US publication US 2008/0280773 A1 and US Ser. No. 13/018,255. The extended nucleic acids can then be fluorescently detected in the presence of scan reagent. Once the fluorescence has been detected, the 3' block may be removed using a deblock reagent that is appropriate to the blocking group used. Exemplary deblock reagents that are useful for respective blocking groups are described in WO04018497, US 2007/0166705A1 and US7057026. The deblock reagent can be washed away leaving target nucleic acids hybridized to extended primers having 3' OH groups that are now competent for addition of a further nucleotide. Accordingly the cycles of adding extension reagent, scan reagent, and deblock reagent, with optional washes between one or more of the steps, can be repeated until a desired sequence is obtained. The above cycles can be carried out using a single extension reagent delivery step per cycle when each of the modified nucleotides has a different label attached thereto, known to correspond to the particular base. The different labels facilitate discrimination between the nucleotides added during each incorporation step. Alternatively, each cycle can include separate steps of extension reagent delivery followed by separate steps of scan reagent delivery and detection, in which case two or more of the nucleotides can have the same label and can be distinguished based on the known order of delivery.

Continuing with the example of nucleic acid clusters in a flow cell, the nucleic acids can be further treated to obtain a second read from the opposite end in a method known as "paired-end sequencing." Paired-end sequencing allows a user to sequence both ends of a target fragment. Paired-end sequencing may facilitate detection of genomic rearrangements and repetitive segments, as well as gene fusions and novel transcripts. Methodology for paired-end sequencing are described in PCT publication WO07010252, PCT application Serial No. PCTGB2007/003798 and US patent application publication US 2009/0088327, each of which is incorporated by reference herein. In one example, a series of steps may be performed as follows; (a) generate clusters of nucleic acids; (b) linearize the nucleic acids; (c) hybridize a first sequencing primer and carry out repeated cycles of extension, scanning and deblocking, as set forth above; (d) "invert' the target nucleic acids on the flow cell surface by synthesizing a complementary copy; (e) linearize the resynthesized strand; and (f) hybridize a second sequencing primer and carry out repeated cycles of extension, scanning and deblocking, as set forth above. The inversion step can be carried out be delivering reagents as set forth above for a single cycle of bridge amplification.

Although the sequencing operation, at 106, has been exemplified above with respect to a particular SBS protocol, it will be understood that other protocols for sequencing any of a variety of other molecular analyses can be carried out as desired. For example, emulsion PCR on beads can also be used, for example as described in Dressman et al., Proc. Natl. Acad. Sci. USA 100:8817-8822 (2003), WO 05/010145, or U.S. Patent Publ. Nos. 2005/0130173 or 2005/0064460. Other sequencing techniques that are applicable for use of the methods and systems set forth herein are pyrosequencing, nanopore sequencing, and sequencing by ligation. Exemplary pyrosequencing techniques and samples that are particularly useful are described in US 6,210,891; US 6,258,568; US 6,274,320 and Ronaghi, Genome Research 11:3-11 (2001. Exemplary nanopore techniques and samples that are also useful are described in Deamer et al., Acc. Chem. Res. 35:817-825 (2002); Li et al., Nat. Mater. 2:611-615 (2003); Soni et al., Clin Chem. 53:1996-2001 (2007) Healy et al., Nanomed. 2:459-481 (2007) and Cockroft et al., J. am. Chem. Soc. 130:818-820; and US 7,001,792. In particular, these methods utilize repeated steps of reagent delivery. An instrument or method set forth herein can be configured with reservoirs, valves, fluidic lines and other fluidic components along with control systems for those components in order to introduce reagents and detect optical signals according to a desired protocol such as those set forth in the references cited above. Any of a variety of samples can be used in these systems such as substrates having beads generated by emulsion PCR, substrates having zero-mode waveguides, substrates having integrated CMOS detectors, substrates having biological nanopores in lipid bilayers, solid-state substrates having synthetic nanopores, and others known in the art. Such samples are described in the context of various sequencing techniques in the references cited above and further in US 2005/0042648; US 2005/0079510; US 2005/0130173; and WO 05/010145.

Systems that may be capable of carrying out one or more of the SBS protocols described above include systems developed by Illumina, Inc., such as the MiSeq, HiSeq 2500, HiSeq X Ten, NeoPrep, HiScan, and iScan systems. Systems capable of carrying out one or more of the SBS protocols described above are described in U.S. Application Nos. 13/273,666 and 13/905,633.

At 108, the sequencing data may be received for subsequent analysis at 110. The sequencing data may include, for example, a number of sample reads. Each sample read may include a sequence of nucleotides, which may be referred to as a sample sequence or a target sequence. The sample sequence may include, fore example, primer sequences, flanking sequences, and a target sequence. The number of nucleotides within the sample sequence may include 30, 40, 50, 60, 70, 80, 90, 100 or more. In some embodiments, one or more the sample reads (or sample sequences) includes at least 150 nucleotides, 200 nucleotides, 300 nucleotides, 400 nucleotides, 500 nucleotides, or more. In some embodiments, the sample reads may include more than 1000 nucleotides, 2000 nucleotides, or more. The sample reads (or the sample sequences) may include primer sequences at one or both ends. In certain embodiments, each sample read may be associated with another read in the opposite direction along the template. For example, the sequencing, at 106, may include paired-end sequencing in which a first read (Read 1) is performed following by a second read (Read 2) in the opposite direction. Each of the first and second reads may include an entirety of a target sequence or nearly an entirety of a target sequence. However, in other embodiments, "asymmetric" paired-end sequencing may be used in which the second read includes only a portion of what can be obtained. For example, the second read may only include a limited number of nucleotides to confirm the identify of the primer sequence that is located near the beginning of the sequence for the second read. By way of example, the first read may include 300-500 nucleotides, but the second read may include only 20-50 nucleotides.

Analyzing, at 110, is described in greater detail below. The analysis, at 110, may include a single protocol or a combination of protocols that analyze the sample reads in a designated manner to obtain desired information. Non-limiting examples of the analysis, at 110, may include analyzing the sample reads to assign the sample reads to (or designate the sample reads for) certain genetic loci; analyzing the sample reads to identify a length and/or sequence of the sample reads; analyzing the sample reads to sort ROIs that are associated with target alleles of a certain locus, analyzing the sample reads (or ROIs) of different target alleles to determine if the ROI of one target allele is suspected stutter product of the ROI of another target allele; identifying a genotype of a genetic locus; and/or monitoring a health or quality control of the assay.

The method 100 may also include generating or providing, at 1 12, a sample report. The sample report may include, for example, information regarding a plurality of genetic loci with respect to the sample. For example, for each genetic locus of a predetermined set of genetic loci, the sample report may at least one of provide a genotype call; indicate that a genotype call cannot be made; provide a confidence score on a certainty of the genotype call; or indicate potential problems with an assay regarding one or more genetic loci. The sample report may also indicate a gender of an individual that provided a sample and/or indicate that the sample include multiple sources. As used herein, a "sample report" may include digital data (e.g., a data file) of a genetic locus or predetermined set of genetic locus and/or a printed report of the genetic locus or the set of genetic loci. Thus, generating or providing, at 112, may include creating a data file and/or printing the sample report, or displaying the sample report.

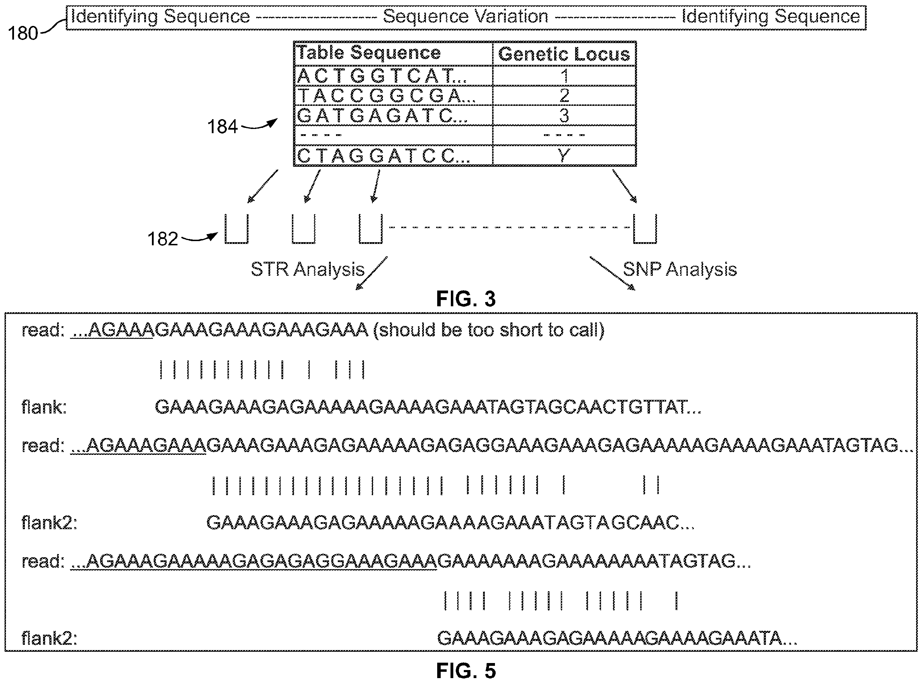

Figure 2 is a flowchart illustrating a method 150 of analyzing sequencing data of sample reads having sequence variations. Figure 2 is described below with reference to Figure 3, which further illustrates the different steps of Figure 1. The method 150 includes receiving, at 152, sequencing data from one or more sources. The sequencing data may include a plurality of sample reads that have corresponding sample sequences of the nucleotides. Figure 3 shows on example of a sample read 180. The terms "identifying sequence" and "sequence variation" represent portions of the sample sequence. Although only one sample read 180 is shown, it should be understood that the sequencing data may include, for example, hundreds, thousands, hundreds of thousands, or millions of sample reads. Different sample reads may have different numbers of nucleotides. For example, a sample read may range between 10 nucleotides to about 500 nucleotides or more. However, the sample reads may include more nucleotides in other embodiments. The sample reads may span the entire genome of the source(s). In particular embodiments, the sample reads are directed toward predetermined genetic loci, such as those genetic loci having suspected STRs or suspected SNPs. The sample reads may be selected based on known primer sequences associated with the genetic loci-of-interest. For example, the sample reads may include PCR amplicons that are obtained using the primer sequences associated with the genetic loci-of-interest.

At 154, each of the sample reads may be assigned to corresponding genetic loci. The sample reads may be assigned to corresponding genetic loci based on the sequence of the nucleotides of the sample read or, in other words, the order of nucleotides within the sample read (e.g., A, C, G, T). Based on this analysis, the sample read may be designated as including a possible allele of a particular genetic locus. The sample read may be collected (or aggregated or binned) with other sample reads that have been designated as including possible alleles of the genetic locus. The different genetic loci are represented as bins 182 in Figure 3. The genetic loci may be a predetermined set of genetic loci that are used for a particular assay. For example, the Federal Bureau of Investigation has identified thirteen (13) STR loci that may be used to generate genetic profiles of possible suspects in crimes. Using the FBI standard as an example, the method 150 may assign each of the sample reads, if possible, to one of the thirteen bins.

The sample reads of different bins may subsequently undergo a different analysis. For example, the sample reads may be assigned to genetic loci that include STRs. Such loci may be referred to as STR loci. However, the sample reads may be assigned to genetic loci that include SNPs. Such loci may be referred to as SNP loci. For a typical sample read, the sample read will be assigned to only one genetic locus (or one bin). In these circumstances, the sample reads will then undergo an analysis that is configured for the type of genetic loci. More specifically, sample reads assigned to an STR locus will undergo an STR analysis, whereas sample reads assigned to an SNP locus will undergo an SNP analysis. In some circumstances, however, it may be possible for a sample read to be assigned to more than one genetic locus and, as such, the sample read may undergo more than one type of analysis.

The assigning operation, at 154, may also be referred to as locus calling in which the sample read is identified as being possibly associated with a particular genetic locus. The sample reads may be analyzed to locate one or more identifying sequences (e.g., primer sequences) of nucleotides that differentiate the sample read from other sample reads. More specifically, the identifying sequence(s) may identify the sample read from other sample reads as being associated with a particular genetic locus. The identifying sequence may include or be located near (e.g., within 10-30 nucleotides) of either end of the sample read. In particular embodiments, the identifying sequences of the sample read are based on primer sequences that were used to selectively amplify sequences from the source or sources. However, in other embodiments, the identifying sequence may not be located near an end of the sample read.

In some embodiments, the identifying sequences are compared to a plurality of predetermined sequences to determine if any of the identifying sequences are identical to or nearly identical to one of the predetermined sequences. For example, each identifying sequence may be compared to a list of predetermined sequences within a database 184 (e.g., look-up table). The predetermined sequences may correlate to certain genetic loci. The predetermined sequences of the database are hereinafter referred to as select sequences. Each select sequence represents a sequence of nucleotides. If an identifying sequence is effectively matched with any of the select sequences, the sample read having the identifying sequence may be assigned to the genetic locus that correlates to the select sequence. It may be possible that a sample read effectively matches more than one select sequence. In such cases, the sample read may be assigned to each of the genetic loci for those select sequences and undergo further analysis to determine which one of the genetic loci that the sample read should be called for.

There may be a predetermined number of select sequences used during the analysis. For example, a genetic profile generated by embodiments set forth herein may include an analysis of from about 5 to about 300 genetic loci. In particular embodiments, the number of genetic loci may be from about 5 to about 100 genetic loci or, more particularly, from about 10 to about 30 genetic loci. However, other numbers of genetic loci may be used. Each genetic locus may have a limited number of select sequences associated with the genetic locus. With a limited number of genetic loci and a limited number of select sequences associated with each genetic locus, the sample reads may be called for genetic loci without an excessive use of computational resources. In some embodiments, the select sequences are based on primer sequences that were used to selectively amplify predetermined sequences of DNA.

Although each select sequence may be based on an identifying sequence of the genetic locus (e.g., primer sequence), the select sequence may not include each and every nucleotide of the identifying sequence. As an example, the select sequence(s) may include a series of *n* nucleotides of the identifying sequence of one of the sample reads. In particular embodiments, the select sequences may include the first n nucleotides of the identifying sequence. The number n may be sufficient to differentiate the alleles of one genetic locus from the alleles of another target locus. In some embodiments, the number n is between 10 and 30.

The assigning operation, at 154, may include analyzing the series of *n* nucleotides of the identifying sequence to determine if the series of *n* nucleotides of the identifying sequence effectively matches with one or more of the select sequences. In particular embodiments, the assigning operation, at 154, may include analyzing the first n nucleotides of the sample sequence to determine if the first *n* nucleotides of the sample sequence effectively matches with one or more of the select sequences. The number *n* may have a variety of values, which may be programmed into the protocol or entered by a user. For example, the number *n* may be defined as the number of nucleotides of the shortest select sequence within the database. The number *n* may be a predetermined number. The predetermined number may be, for example, 10, 11, 12, 13, 14, 15, 16, 17, 18, 19, 20, 21, 22, 23, 24, 25, 26, 27, 28, 29, or 30 nucleotides. However, fewer or more nucleotides may be used in other embodiments. The number *n* may also be selected by an individual, such as a user of the system. The number *n* may be based on one or more conditions. For instance, the number *n* may be defined as the number of nucleotides of the shortest primer sequence within the database or a designated number, which ever is the smaller number. In some embodiments, a minimum value for *n* may be used, such as 15, such that any primer sequence that is less than 15 nucleotides may be designated as an exception.

In some cases, the series of *n* nucleotides of an indentifying sequence may not precisely match the nucleotides of the select sequence. Nonetheless, the identifying sequence may effectively match the select sequence if the identifying sequence is nearly identical to the select sequence. For example, the sample read may be called for a genetic locus if the series of *n* nucleotides (e.g., the first *n* nucleotides) of the identifying sequence match a select sequence with no more than a designated number of mismatches (e.g., 3) and/or a designated number of shifts (e.g., 2). Rules may be established such that each mismatch or shift may count as a difference between the sample read and the primer sequence. If the number of differences is less than a designated number, then the sample read may be called for the corresponding genetic locus (i.e., assigned to the corresponding genetic locus). In some embodiments, a matching score may be determined that is based on the number of differences between the identifying sequence of the sample read and the select sequence associated with a genetic locus. If the matching score passes a designated matching threshold, then the genetic locus that corresponds to the select sequence may be designated as a potential locus for the sample read. In some embodiments, subsequent analysis may be performed to determine whether the sample read is called for the genetic locus.

The designated number of differences between the identifying sequence and the select sequence may be, for example, a number that is less than 20% of the total number of nucleotides within the corresponding select sequence, or, more specifically, a number less than 15% of the total number of nucleotides within the corresponding select sequence. The designated number of differences may be a predetermined value, such as be 6, 5, 4, 3, or 2. Accordingly, the phrase "effectively matches" includes the sample sequence having a series of *n* nucleotides that exactly matches a select sequence or nearly matches a select sequence with a limited number of differences between the select sequence and the series of *n* nucleotides.

If the sample read effectively matches one of the select sequences in the database (i.e., exactly matches or nearly matches as described above), then the sample read is assigned or designated to the genetic locus that correlates to the select sequence. This may be referred to as locus calling or provisional-locus calling, wherein the sample read is called for the genetic locus that correlates to the select sequence. However, as discussed above, a sample read may be called for more than one genetic locus. In such embodiments, further analysis may be performed to call or assign the sample read for only one of the potential genetic loci.

In some embodiments, the sample read that is compared to the database is the first read from paired-end sequencing. For more particular embodiments, the second read that correlates to the sample read may be analyzed to confirm that an identifying sequence within the second read effectively matches a select sequence from the database. The select sequences in the database for the second reads may be different than the select sequences used for the first reads. In some embodiments, the sample read is called for the genetic locus only after confirming that the second read also effectively matches with a select sequence in the database. Determining whether the second read effectively matches a select sequence may be performed in a similar manner as described above. By confirming that the second read effectively matches a select sequence, off-target sample reads (e.g., off-target amplicons) may be filtered from further analysis.

The sample reads that have been called for a particular genetic locus may be referred to as "assigned reads" of the particular genetic locus. At this stage, although the assigned reads have been identified as possibly correlating to a particular genetic locus, it is possible that the assigned read will not be suitable for further analysis. More specifically, an assigned read (or reads) may be subsequently removed from further analysis based on other factors.

After assigning, at 154, the assigned reads to corresponding genetic loci, the sample reads may then be further analyzed. The subsequent analysis that is performed with the assigned reads may be based on the type of genetic locus that has been called for the assigned read. For example, if a genetic locus is known for including SNPs, then the assigned reads that have been called for the genetic locus may undergo analysis, at 156, to identify the SNPs of the assigned reads. If the genetic locus is known for including polymorphic repetitive DNA elements, then the assigned reads may be analyzed, at 158, to identify or characterize the polymorphic repetitive DNA elements within the sample reads. In some embodiments, if an assigned read effectively matches with an STR locus and an SNP locus, a warning or flag may be assigned to the sample read. The sample read may be designated as both an STR locus and an SNP locus an undergo, for example, analysis at 156 and analysis at 158.

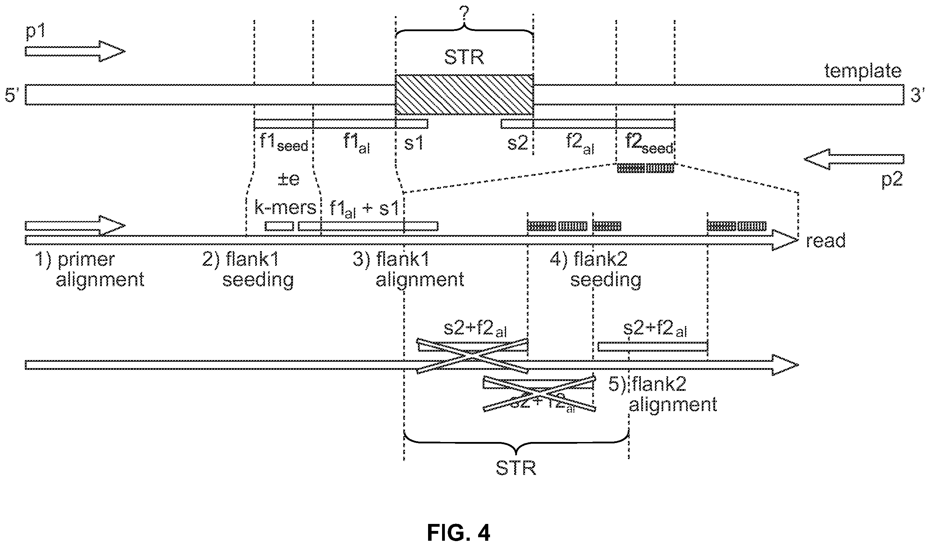

In some embodiments, the STR analysis may be executed using the protocol described below with respect to Figures 4-7. The analysis at 158 may include analyzing the sample reads to identify ROIs, which may include determining sequences of the ROIs and/or lengths of the ROIs. The ROIs may be sequences of the sample reads (e.g., sub-sequences of sample sequences). The ROIs may include repetitive segments. The ROIs may be sequences of nucleotides that only include one or more series of repeat motifs (i.e., the repetitive segment) or include the one or more series of repeat motifs in addition to a designated number of nucleotides extending from one or both ends of the repetitive segment. More specifically, each of the ROIs may include one or more series of repeat motifs in which each repeat motif includes an identical set of nucleotides (e.g., two, three, four, five, six nucleotides, or more) of nucleotides. Commonly used repeat motifs include tetranucleotides, but other motifs may be used, such as mono-, di-, tri-, penta-, or hexanucleotides. In particular embodiments, the repeat motifs include tetranucleotide.

The analysis, at 158, may include analyzing the assigned reads for each designated locus to identify corresponding ROIs within the assigned reads. More specifically, a length and/or sequence of the ROIs may be determined. The analyzing, at 158, may include aligning the assigned reads in accordance with an alignment protocol to determine sequences and/or lengths of the assigned reads. The alignment protocol may include the method described in International Application No. PCT/US2013/030867 (Publication No. WO 2014/142831), filed on March 15, 2013.

Other alignment protocols, however, may be used. For example, one known alignment protocol aligns a sample read to a reference sequence. Another existing approach aligns the sample read to a reference ladder. In this example, a "reference genome" is created by building a ladder of all known STR alleles and aligning the reads to the reference genome, as typically done with NGS whole genome sequence data or targeted sequencing of non-repetitive DNA regions. Another methodology that may be used with embodiments set forth herein is known as lobSTR. The lobSTR method senses then calls all existing STRs from sequencing data of a single sample *de novo,* with no prior knowledge of the STRs (*see* Gymrek et al. 2012 Genome Research 22:1154-62).

The alignment method set forth in International Application No. PCT/US2013/030867 (Publication No. WO 2014/142831) is now described for genetic loci that include ROIs. For ease of reading, such genetic loci may be referred to as STR loci. In some embodiments, the conserved flanks of STR loci are used to effectively determine the sequence of the repetitive segment. After assigning, at 154, the sample reads to corresponding STR loci, embodiments may align sections of flanking sequences on each side of the corresponding repetitive segment to determine a length and sequence of the repetitive segment. The alignments may be seeded using a k-mer strategy. The seed regions can be, for example, in a selected high-complexity region of the flanking sequence, close to the repetitive segment, but avoiding low-complexity sequence with homology to the repetitive segment. Such an approach may avoid misalignment of low-complexity flanking sequences close to the repetitive segment.

Embodiments may utilize known sequences in the flanks of the STR themselves, which have been previously defined based on the known existing variations among the human population. Advantageously, performing alignment of a short span of flanking region is computationally quicker than other methods. For example, a dynamic programming alignment (Smith-Waterman type) of the entire read can be CPU intensive, time consuming, especially where multiple sample sequences are to be aligned. Furthermore, time spent aligning an entire sequence (for which a reference may not even exist) takes up valuable computational resources.

Embodiments may utilize prior knowledge of a flanking sequence to ensure the proper call of the STR allele. In contrast, existing methods, which rely on a full reference sequence for each allele, face significant failure rates in situations where there is an incomplete reference. There are many alleles for which the sequence is not known, and possibly some yet unknown alleles. By way of illustration, assume a repetitive segment with a simple repeat motif [TCTA] having a 3' flank starting with the sequence TCAGCTA. Thus, the reference may include such sequences as [flank1][TCTA]ₙTCAGCTA[rest_of_flank2], where "n" is the number of repeats in the allele. The 9.3 allele would differ from the 10 allele by having a deletion somewhere along the sequence. These may be included in the reference, though it could be that not all are. [TCTA]₇TCA[TCTA]₂ is an example of such an allele. Under existing alignment protocols, any read ending after the [TCTA]₇ and before the final [TCTA], will align to [flank1][TCTA]₇TCAGCTA, making an improper call.

Embodiments provided herein allow for determining the length of a polymorphic repetitive DNA element or a repetitive segment situated between a first conserved flanking region and a second conserved flanking region. In one embodiment, the methods comprise providing a data set comprising at least one sample read of a polymorphic repetitive DNA element; providing a reference sequence comprising the first conserved flanking region and the second conserved flanking region; aligning a portion of the first flanking region of the reference sequence to the sample read; aligning a portion of the second flanking region of the reference sequence to the sample read; and determining the length and/or sequence of the repetitive segment. In typical embodiments, one or more steps in the method are performed using a suitably programmed computer.

As used herein, the term "sample read" refers to sequence data for which the length and/or identity of the repetitive element are to be determined. The sample read may be based on DNA or RNA. The sample read can comprise all of the repetitive element, or a portion thereof. The sample read can further comprise a conserved flanking region on one end of the repetitive element (e.g., a 5' flanking region). The sample read can further comprise an additional conserved flanking region on another end of the repetitive element (e.g., a 3' flanking region). In typical embodiments, the sample read comprises sequence data from a PCR amplicon having a forward and reverse primer sequence. The sequence data can be obtained from any select sequence methodology. The sample read can be, for example, from a sequencing-by-synthesis (SBS) reaction, a sequencing-by-ligation reaction, or any other suitable sequencing methodology for which it is desired to determine the length and/or identity of a repetitive element. The sample read can be a consensus (e.g., averaged or weighted) sequence derived from multiple sample reads. In certain embodiments, providing a reference sequence comprises identifying a locus-of-interest based upon the primer sequence of the PCR amplicon.

As used herein, the term "polymorphic repetitive DNA element" refers to any repeating DNA sequence, which may be referred to as a repetitive segment. Methods provided herein can be used to align the corresponding flanking regions of any such repeating DNA sequence. The methods presented herein can be used for any region which is difficult to align, regardless of the repeat class. The method presented herein are especially useful for a region having conserved flanking regions. Additionally or alternatively, the methods presented herein are especially useful for sample reads which span the entire repetitive segment including at least a portion of each flanking region. In typical embodiments, the repetitive DNA element is a variable number tandem repeat (VNTR). VNTRs are polymorphisms where a particular sequence is repeated at that locus numerous times. Some VNTRs include minisatellites, and microsatellites, also known as simple sequence repeats (SSRs) or short tandem repeats (STRs). In some embodiments, the repetitive segment is less than 100 nucleotides, although larger repetitive segments can be aligned. The repeating unit (e.g., repeat motif) of the repetitive segment can be 2, 3, 4, 5, 6, 7, 8, 9, 10, 11, 12, 13, 14, 15, 16, 17, 18, 19, 20 or more nucleotides, and can be repeated up to 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 55, 60, 65, 70, 75, 80, 85, 90, 95 or up to at least 100 times or more.

In certain embodiments, the polymorphic repetitive DNA element is an STR. In some embodiments, the STR is used for forensic purposes. In typical embodiments for forensic applications, for example, the polymorphic repetitive DNA element comprises tetra- or penta-nucleotide repeat motifs, however, the methods provided herein are suitable for any length of repeat motif. In certain embodiments, the repetitive segment is a short tandem repeat (STR) such as, for example, a STR selected from the CODIS autosomal STR loci, CODIS Y-STR loci, EU autosomal STR loci, EU Y-STR loci and the like. As an example, the CODIS (Combined DNA Index System) database is a set of core STR loci for identified by the FBI laboratory and includes 13 loci: CSF1PO, FGA, TH01, TPOX, VWA, D3S1358, D5S818, D7S820, D8S1179, D13S317, D16S539, D18S51 and D21S11. Additional STRs of interest to the forensic community and which can be aligned using the methods and systems provided herein include PENTA D and PENTA E. The methods and systems presented herein can be applied to any repetitive DNA element and are not limited to the STRs described above.

As used herein, the term "reference sequence" refers to a known sequence which acts as a scaffold against which a sample sequence can be aligned. In typical embodiments of the methods and systems provided herein, the reference sequence comprises at least a first conserved flanking region and a second conserved flanking region. The term "conserved flanking region" refers to a region of sequence outside the repetitive segment (e.g., STR). The region is typically conserved across many alleles, even though the repetitive segment may be polymorphic. A conserved flanking region as used herein typically will be of higher complexity than the repetitive segment. In typical embodiments, a single reference sequence can be used to align all alleles within a genetic locus. In some embodiments, more than one reference sequence is used to align sample sequences of a genetic locus because of variation within the flanking region. For example, the repetitive segment for Amelogenin has differences in the flanks between X and Y, although a single reference can represent the repetitive segment if a longer region is included in the reference.

In embodiments presented herein, a portion of a flanking region of a reference sequence is aligned to the sample sequence. Aligning is performed by determining a location of the conserved flanking region and then conducting a sequence alignment of that portion of the flanking region with the corresponding portion of the sample read. Aligning of a portion of a flanking region is performed according to known alignment methods. In certain embodiments, the aligning of a portion of the flanking region (e.g., the first or second flanking regions) includes: (i) determining a location of a conserved flanking region on the sample read by using exact k-mer matching of a seeding region which overlaps or is adjacent to the repetitive segment; and (ii) aligning the flanking region to the sample read. In some embodiments, the aligning can further comprise aligning both the flanking sequence and a short adjacent region comprising a portion of the repetitive segment.

An example of this approach is illustrated in Figure 4. An amplicon ("template"), which may also be referred to as the sample read, is shown in Figure 4 having a STR of unknown length and/or identity. As described above with respect to Figure 2, the sample read may be analyzed to assign the sample read to a genetic locus, which is known to include an STR in this case. After determining the genetic locus for the sample read, the alignment protocol may include aligning a predetermined sequence of the sample read with a predetermined sequence of the reference sequence. For example, the primers are illustrated as p1 and p2, which are based on the primer sequences that were used to generate the amplicon. In the embodiment shown in Figure 4, p1 alone is used during the initial alignment step. In some embodiments, p2 alone is used for primer alignment. In other embodiments, both p1 and p2 are used for primer alignment. Yet in other embodiments, other sequences may be used for the initial alignment step.

Following the initial alignment, flank 1 is aligned, designated in Figure 4 as "f1ₐ₁." Flank 1 alignment can be preceded by seeding of flank 1, designated in Figure 4 as "f1_{seed}." Flank 1 seeding is to correct for a small number (designated as "e") of indels between the beginning of the sample sequence and the STR. The seeding region may be directly next to the beginning of the STR, or may be offset (as in figure) to avoid low-complexity regions. Seeding can be done by exact k-mer matching. Flank 1 alignment proceeds to determine the beginning position of the STR sequence. If the STR pattern is conserved enough to predict the first few nucleotides (s1), these are added to the alignment for improved accuracy.

Since the length of the STR is unknown, an alignment is performed for flank 2 as follows. Flank 2 seeding is performed to quickly find out possible end positions of the STR. As the seeding for flank 1, the seeding may be offset to avoid low-complexity regions and mis-alignment. Any flank 2 seeds that fail to align are discarded. Once flank 2 properly aligns, the end position (s2) of the STR can be determined. With the beginning of the STR sequence known at s1 and the end of the STR sequence known with at s2, a length of the STR can be calculated.

The seeding region can be directly adjacent to the repetitive segment (e.g., the STR) and/or comprises a portion of the repetitive segment. In some embodiments, the location of the seeding region will depend on the complexity of the region directly adjacent to the repetitive segment. The beginning or end of an STR may be bounded by sequences that comprises additional repeats or which has low complexity. Thus, it can be advantageous to offset the seeding of the flanking region in order to avoid regions of low complexity. As used herein, the term "low-complexity" refers to a region with sequence that resembles that of the repeat motifs and/or repetitive segment. Additionally or alternatively, a low-complexity region incorporates a low diversity of nucleotides. For example, in some embodiments, a low-complexity region comprises sequence having more than 30%, 40%, 50%, 60%, 70% or more than 80% sequence identity to the repeat sequence. In typical embodiments, the low-complexity region incorporates each of the four nucleotides at a frequency of less than 20%, 15%, 10% or less than 5% of all the nucleotides in the region. Any suitable method may be utilized to determine a region of low-complexity. Methods of determining a region of low-complexity are known in the art, as exemplified by the methods disclosed in Morgulis et al., (2006) Bioinformatics. 22(2):134-41, y. For example, as described in the materials for Morgulis et al., an algorithm such as DUST may be used to identify regions within a given nucleotide sequence that have low complexity.

In some embodiments, the seeding is offset from a beginning of the STR by at least 1, 2, 3, 4, 5, 6, 7, 8, 9, 10, 15, 20, 25, 30, 35, 40 or more nucleotides. In some embodiments, the flanking region is evaluated to identify a region of high complexity. As used herein, the term "high-complexity region" refers to a region with sequence that is different enough from that of repeat motif and/or repetitive segment that it reduces the likelihood of mis-alignments. Additionally or alternatively, a high complexity region incorporates a variety of nucleotides. For example, in some embodiments, a high-complexity region comprises sequence having less than 80%, 70%, 60%, 50%, 40%, 30%, 20% or less than 10% identity to the repeat sequence. In typical embodiments, the high-complexity region incorporates each of the four nucleotides at a frequency of at least 10%, 15%, 20%, or at least 25% of all the nucleotides in the region.

As used herein, the term "exact *k* -mer matching" refers to a method to find optimal alignment by using a word method where the word length is defined as having a value *k.* In some embodiments, the value of k is 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 or more nucleotides in length. In typical embodiments, *k* has a value of between 5 and 30 nucleotides in length. In some embodiments, *k* has a value of between 5 and 16 nucleotides in length. In certain embodiments, *k* is selected by the system or user based on one or more factors. For example, if a flank region is short, such as when the primer sequence is located relatively close to the STR sequence, *k* may be reduced appropriately. In typical embodiments, *k* is chosen so as to find all matches within a distance of +/e.

Word methods identify a series of short, non-overlapping subsequences ("words") in the query sequence that are then matched to candidate database sequences. The relative positions of the word in the two sequences being compared are subtracted to obtain an offset; this will indicate a region of alignment if multiple distinct words produce the same offset. Only if this region is detected do these methods apply more sensitive alignment criteria; thus, many unnecessary comparisons with sequences of no appreciable similarity are eliminated. Methods of performing k-mer matching, including exact k-mer matching, are known in the art, as exemplified by the disclosure of Lipman, et al., (1985) Science 227:1435-41, and of Altschul, et al., (1990) Journal of Molecular Biology 215:403-410.

As used herein, the term "amplicon" refers to any suitable amplification product for which a sequence is obtained. Typically, the amplification product is a product of a selective amplification methodology, using target-specific primers, such as PCR primers. In certain embodiments, the sequence data is from a PCR amplicon having a forward and reverse primer sequences. In some embodiments, the selective amplification methodology can include one or more non-selective amplification steps. For example, an amplification process using random or degenerate primers can be followed by one or more cycles of amplification using target-specific primers. Suitable methods for selective amplification include, but are not limited to, the polymerase chain reaction (PCR), strand displacement amplification (SDA), transcription mediated amplification (TMA) and nucleic acid sequence based amplification (NASBA), as described in U.S. Pat. No. 8,003,354. The above amplification methods can be employed to selectively amplify one or more nucleic acids of interest. For example, PCR, including multiplex PCR, SDA, TMA, NASBA and the like can be utilized to selectively amplify one or more nucleic acids of interest. In such embodiments, primers directed specifically to the nucleic acid of interest are included in the amplification reaction. Other suitable methods for amplification of nucleic acids can include oligonucleotide extension and ligation, rolling circle amplification (RCA) (Lizardi et al., Nat. Genet. 19:225-232 (1998)) and oligonucleotide ligation assay (OLA) (See generally U.S. Pat. Nos. 7,582,420, 5,185,243, 5,679,524 and 5,573,907; EP 0 320 308 B1; EP 0 336 731 B1; EP 0 439 182 B1; WO 90/01069; WO 89/12696; and WO 89/09835.

It will be appreciated that these amplification methodologies can be designed to selectively amplify a target nucleic acid of interest. For example, in some embodiments, the selective amplification method can include ligation probe amplification or oligonucleotide ligation assay (OLA) reactions that contain primers directed specifically to the nucleic acid of interest. In some embodiments, the selective amplification method can include a primer extension-ligation reaction that contains primers directed specifically to the nucleic acid of interest. As a non-limiting example of primer extension and ligation primers that can be specifically designed to amplify a nucleic acid of interest, the amplification can include primers used for the GoldenGate^{™} assay (Illumina, Inc., San Diego, CA), as described in U.S. Pat. No. 7,582,420. The present methods are not limited to any particular amplification technique and amplification techniques described herein are exemplary only with regard to methods and embodiments of the present disclosure.

Primers for amplification of a repetitive DNA element typically hybridize to the unique sequences of flanking regions. Primers can be designed and generated according to any suitable methodology. Design of primers for flanking regions of repetitive segments is well known in the art, as exemplified by Zhi, et al. (2006) Genome Biol, 7(1):R7. For example, primers can be designed manually. This involves searching the genomic DNA sequence for microsatellite repeats, which can be done by eye or by using automated tools such as RepeatMasker software. Once the repetitive segments and the corresponding flanking regions are determined, the flanking sequences can be used to design oligonucleotide primers which will amplify the specific repeat in a PCR reaction.

The following describes examples that have been performed in accordance with the above description.

### EXAMPLE 1 - Alignment of the locus D18S51

This example describes alignment of the locus D18S51 according to one embodiment. Some loci have flanking sequences which are low-complexity and resemble the STR repeat sequence. This can cause the flanking sequence to be mis-aligned (sometimes to the STR sequence itself) and thus the allele can be mis-called. An example of a troublesome locus is D18S51. The repeat motif is [AGAA] n AAAG AGAGAG. The flanking sequence is shown below with the low-complexity "problem" sequence underlined:

### GAGACCTTGTCTC (STR) GAAAGAAAGAGAAAAAGAAAAGAAATAGTAGCAACTGTTAT

If the flanking region immediately adjacent to the STR were used to seed the alignment, k-mers would be generated such as GAAAG, AAAGAA, AGAGAAA, which map to the STR sequence. This deters performance since many possibilities are obtained from the seeding, but most importantly, the approach creates mis-alignments, such as those shown in Figure 5. In the sequences shown in Figure 5, the true STR sequence is highlighted, the STR sequence resulting from the mis-alignment is underlined and read errors are shown in bold.

For these low-complexity flanks, it was ensured that the seeding regions are not in the low-complexity region by pushing them further away from the STR sequence. While this requires longer reads to call the STR, it ensures high-accuracy and prevents mis-alignment of the flanking region to STR sequence (or other parts of the flank). The low-complexity flank is still aligned to the read to find the ending position of the STR but because the alignment is seeded with high-complexity sequence it should be in the correct position.

### EXAMPLE 2 - Alignment of the locus Penta-D by short STR Sequence Addition

A set of Penta-D sequences tended to have STRs that were 1 nt shorter than expected. Upon further inspection, it was discovered that both flanks contained poly-A stretches and sequencing / amplification errors often removed one of the A's in those stretches. As shown in the sequence below, homopolymeric A stretches are found on both flanks.

| | | |
|---|---|---|
| ...CAAGAAAG**AAAAAAAA**G | **[AAAGA]ₙ** | **AAAAA**CGAAGGGG**AAAAAAA**GAGAAT... |

A read error causing a deletion in the first flank would yield to two equally viable alignments:

| | | |
|---|---|---|
| read: | ...CAAGAAAG**AAAAAAA**-GA... | |
| flank: | ...CAAGAAAG**AAAAAAAA**G- | (2 indels) |
| read: | ...CAAGAAAG**AAAAAAA**GA... | (2 mismatches) |
| flank: | ...CAAGAAAG**AAAAAAAA**G | |

Enforcing the base closest to the STR to be a match did not work because one of the flanks in one of the STRs ended up having a SNP in it. It was discovered that adding just 2 nucleotides of the STR sequence solved the issue:

| | | |
|---|---|---|
| read: | ... CAAGAAAG**AAAAAAA**-GAA | |
| flank: | ... CAAGAAAG**AAAAAAAA**GAA | (1 indel) ✔ |
| read: | ... CAAGAAAG**AAAAAAA**G-AA | (1 indel + 1 mismatch) |
| flank: | ...CAAGAAAG**AAAAAAAA**GAA | |

### EXAMPLE 3 - Analysis of Mixture of DNA Samples

A mixture of samples was analyzed using the methods provided herein to make calls for each locus in a panel of forensic STRs. For each locus, the number reads corresponding to each allele and to each different sequence for that allele were counted.

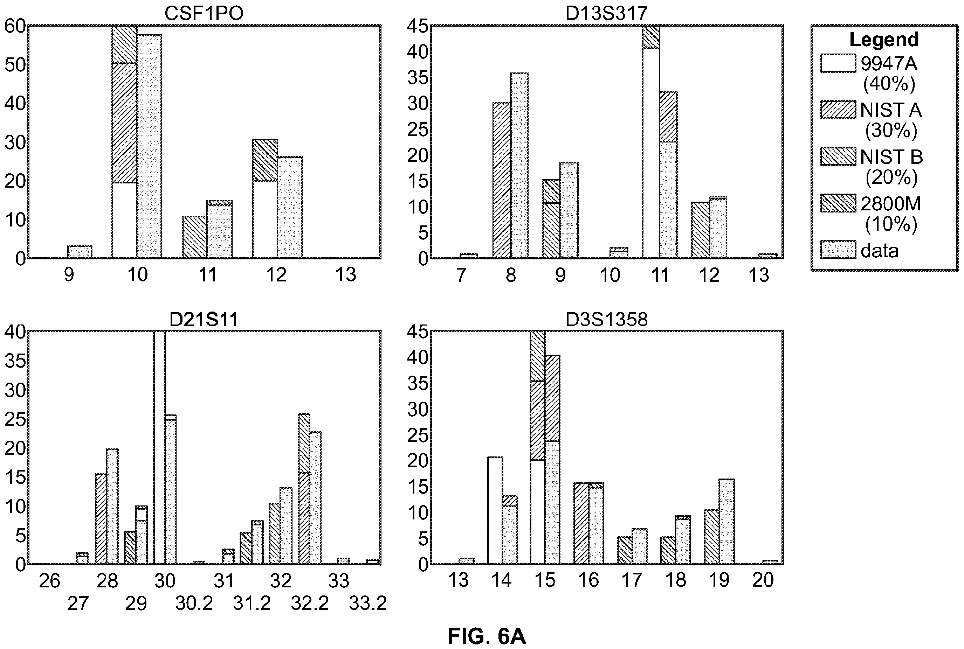

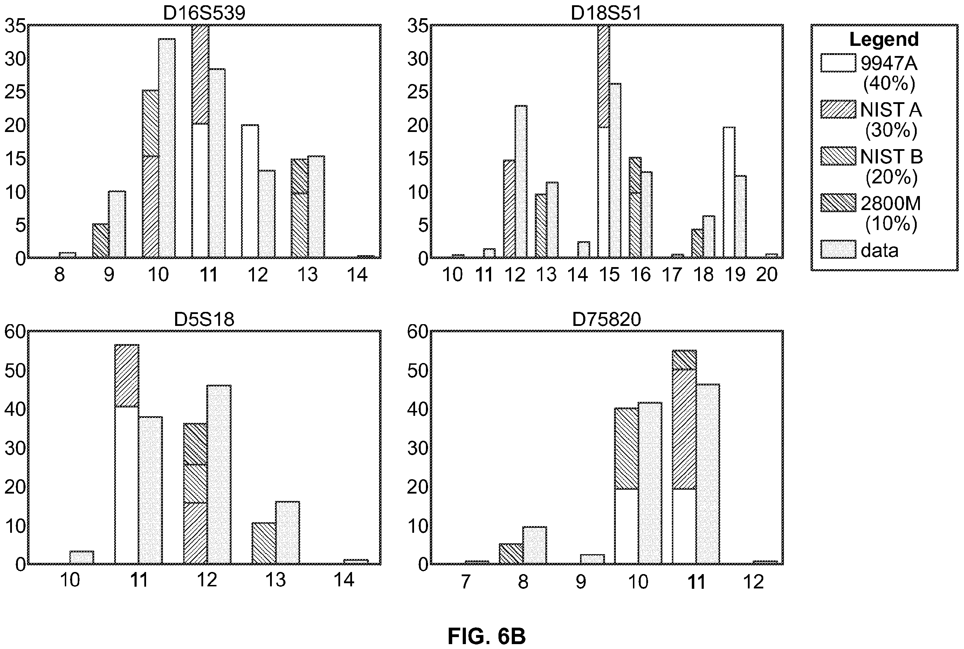

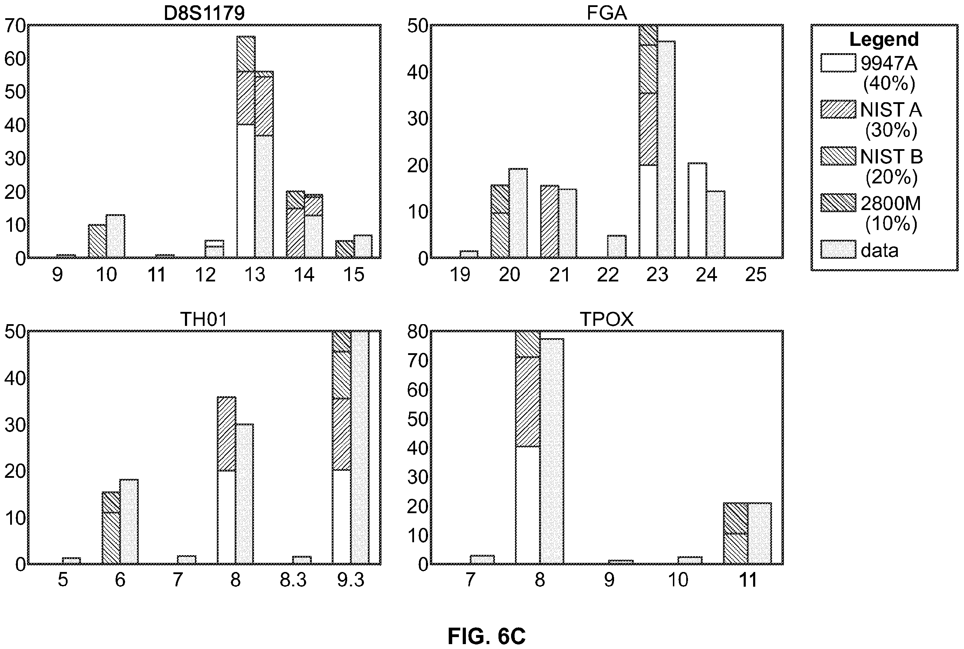

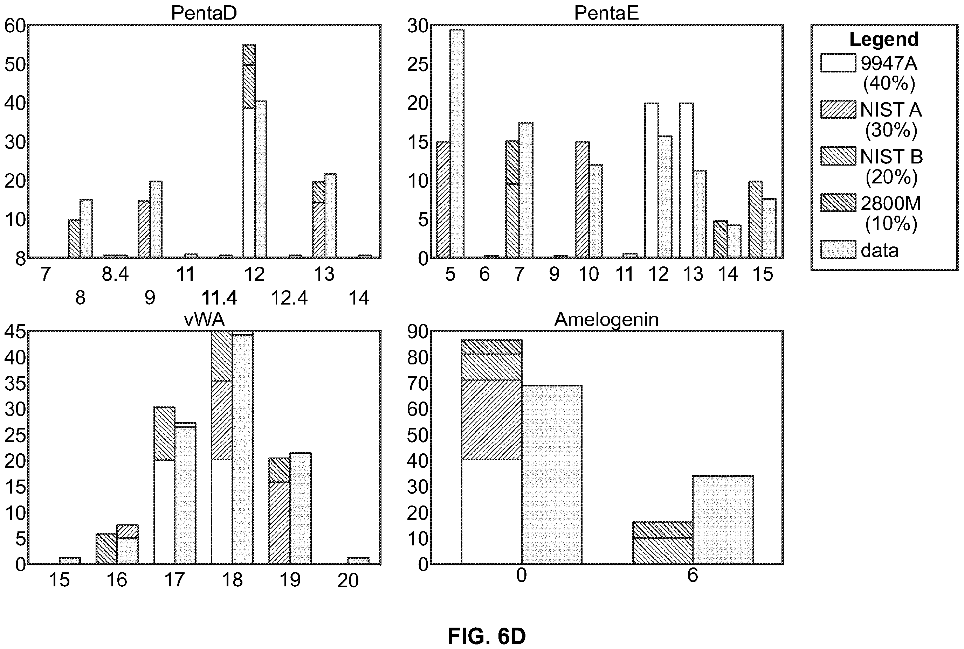

Typical results are shown in Figures 6A-6D. As shown, the bar on the right of each pair represents the actual data obtained, indicating the proportion of reads for each allele. Different shades represent different sequences. Alleles with less than 0.1% of the locus read count and sequences with less than 1% of the allele count are omitted. The bar on the left side of each pair represents the theoretical proportions (no stutter). Different shades represent different control DNA in the input as indicated in the legend. In Figure 6A-6D, the x-axis is in order allele, and the Y axis indicates proportion of reads with the indicated allele.

As shown in the Figure, the STR calling approach using the methods presented herein achieved surprisingly accurate calls for each allele in the panel.

### EXAMPLE 4 - Analysis of Forensic STR Panel

A panel of 15 different loci were analyzed in 5 different samples. The samples were obtained from Promega Corp, and included samples 9947A, K562, 2800M, NIST: A and B (SRM 2391c). The loci were chosen from the CODIS STR forensic markers and included CSF1PO, D3S1358, D7S820, D16S539, D18S51, FGA, PentaE, TH01, vWA, D5S818, D8S1179, D13S317, D21S11, PentaD and TPOX using the alignment method presented herein. Briefly, the markers were amplified using standard primers, as set forth in Krenke, et al. (2002) J. Forensic Sci. 47(4): 773-785. The amplicons were pooled and sequencing data was obtained using 1x460 cycles on a MiSeq sequencing instrument (Illumina, San Diego, CA).

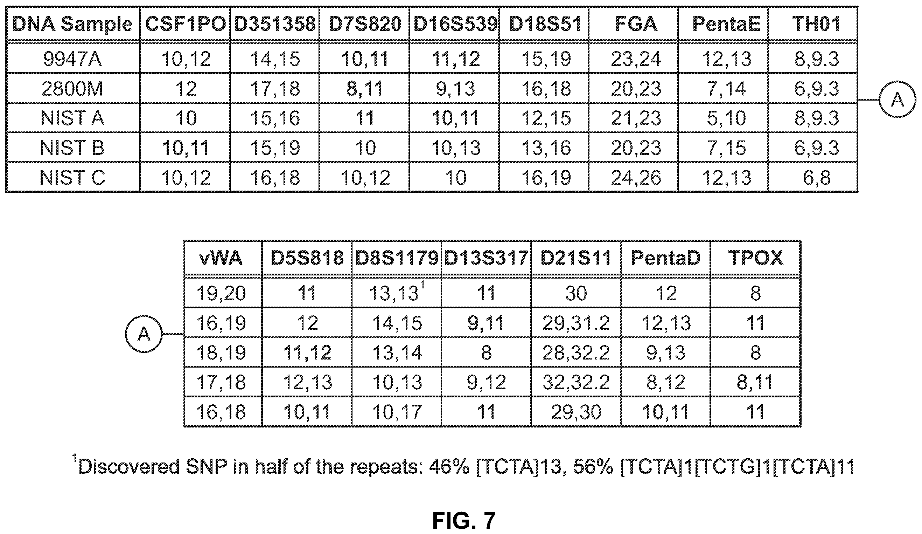

Alignment was performed according to the methods presented herein. As set forth in Figure 7, 100% concordance for these control samples was shown compared to control data. In addition, this method identified a previously-unknown SNP in one of the samples for marker D8S1179, further demonstrating the powerful tool of sequence-based STR analysis when combined with the alignment methods provided herein.

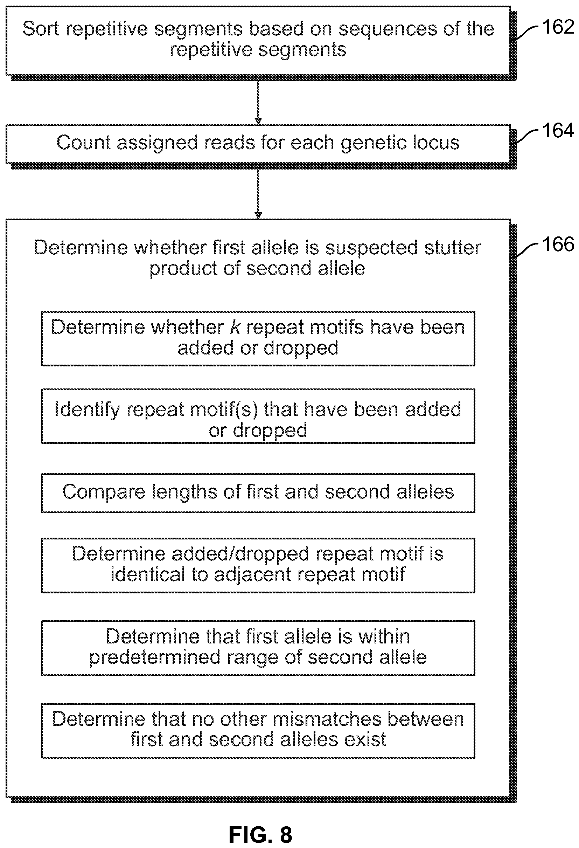

Figure 8 illustrates a method 160 of identifying stutter product. After the ROIs within the assigned reads have been identified, embodiments set forth herein may sort, at 162, the ROIs (or the assigned reads) based on the sequences of the ROIs. As described above, in certain circumstances, the alignment protocol may analyze a portion of one or both of the flanking regions in addition to the sequence of the repetitive segment. Accordingly, in certain embodiments, the sorting, at 162, may include sorting based on the sequence of the repetitive segment and a sub-sequence of one or both of the flanking regions. As an example, the sorting may include analyzing the repetitive segment and a few nucleotides of each of the flanking regions that extend from the repetitive segment. In other embodiments, the sorting, at 162, may include sorting that is based on an ROI that only includes sequence of the repetitive segment.

The ROIs (or repetitive segments) may be sorted such that the ROIs (or repetitive segments) with different sequences are designated as being potential (or suspected) alleles. For example, each potential allele may have a unique sample sequence and/or a unique length. More specifically, each potential allele may have a unique sequence of the ROI or repetitive segment and/or a unique length of the ROI or repetitive segment. As described below, in some embodiments, the repetitive segments may be ordered based on CE Allele name.

The sorting, at 162, may be performed for each designated locus. After the sample reads are assigned to corresponding genetic loci, each genetic locus may have a plurality of assigned reads associated with the genetic locus. For example, in some embodiments, one or more the genetic loci may have hundreds of assigned reads that are grouped or binned with each other. As is known, a corresponding genetic locus, such as a known STR locus, may have a plurality of alleles in which each allele includes a different sequence. By collectively analyzing the plurality of assigned reads that have been identified as being from a common genetic locus, the plurality of assigned reads may be analyzed to provide a genotype call for an individual or plurality of individuals.

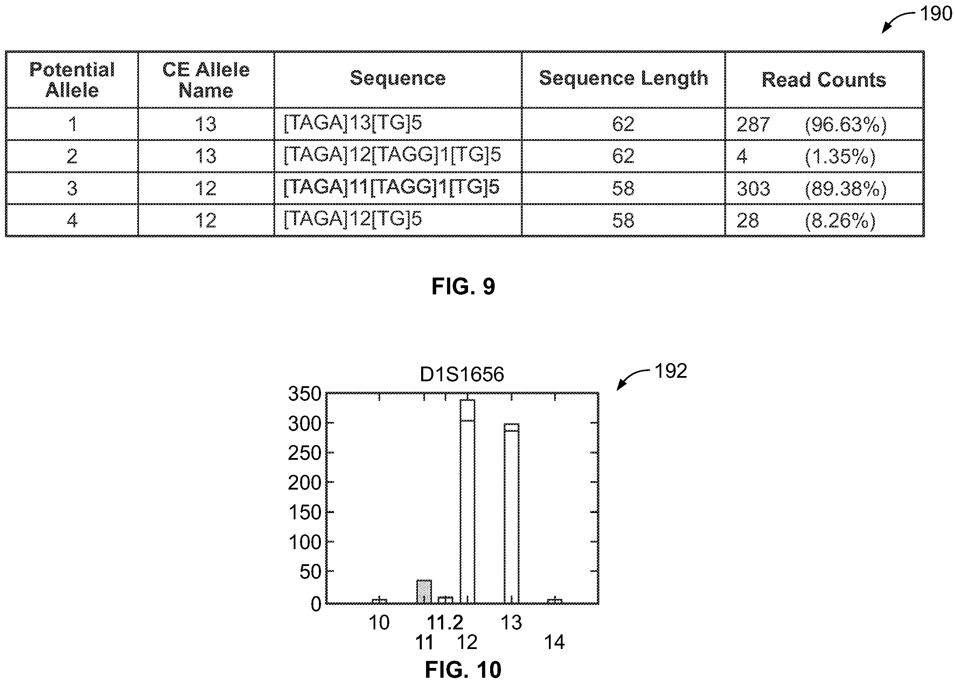

The method 160 may also include counting (or summing), at 164, the assigned reads of a common genetic locus that have a common sequence. The counting, at 164, may include determining count scores as described herein. By way of example, Figure 9 includes a table 190 that includes the potential alleles for D1S1656 locus, and Figure 10 includes a graph 192 that illustrates the distribution of the CE alleles. CE alleles are named in accordance with convention and, as shown in Figure 10, possibly include stutter product. In this example, after sequencing nucleic acids from a single source, the sample reads were analyzed to identify ROIs (e.g., repetitive segments) for the D1S1656 locus. The ROIs were sorted and counted to identify a number of potential alleles within the D1S1656 locus. In this example, alleles having counts that were below 1% of the total number of assigned reads of the D1S1656 locus were not considered. As shown in Figure 9, the filtered assigned reads included a total of four unique sequences, which may be considered potential alleles of the D1S1656 locus. After analysis, as described below, the genotype call for the locus is heterozygous 12/13.

In some embodiments, based on the count scores of the potential alleles of a genetic locus, a genotype call can be made for the genetic locus. In some embodiments, however, further analysis of the sequences may be performed. For example, the method 160 may include analyzing, at 166, the sequences of the potential alleles to determine whether a first allele is a suspected stutter product of a second allele. Stutter is a phenomenon that may occur during amplification of a nucleic acid, especially nucleic acids that include one or more series of repeat motifs, such as those found within STR alleles. Stutter products have sequences that are typically one or more repeat motifs less in size (or more in size) than the true allele. During replication of a nucleic acid sequence, two strands may come apart along the STR. Since each repeat motif is the same, the two strands may re-anneal improperly such that the two strands are off-set by one or more repeat motifs. Thus, the resulting product, which may be further amplified, differs from the true sequence by one or more repeat motifs.

Because stutter products are nearly the same size as the true allele, it can be challenging to determine whether a stutter product is a true allele of the genetic locus or a stutter product of an adjacent allele. Accordingly, the stutter product can reduce the confidence of a genotype call. Under certain circumstances, the stutter product may prevent a genotype call from being provided or potentially cause an incorrect genotype call. Stutter product can render genotype calls for samples that include a plurality of sources especially challenging.

The analyzing, at 166, may determine whether a first allele is a suspected stutter product of a second allele. In some embodiments, the analysis includes applying one or more rules or conditions to the sequences of the first and second alleles. For example, the first allele may be a suspected stutter product of the second allele if it is determined, at 171, that *k* repeat motifs have been added or dropped between the first and second alleles. The number *k* is a whole number. In particular embodiments, the number *k* is 1 or 2. Although stutter product typically includes one less repeat motif, stutter product may also include two less repeat motifs or one added repeat motif. It is possible that stutter product also include other differences in repeat motifs. The analyzing, at 166, may include comparing each potential allele associated with a genetic locus to each other potential allele of the same genetic locus.

In some embodiments, the analysis, at 166, may include identifying, at 172, the repeat motif(s) that have been added or dropped. Identifying, at 172, the repeat motifs that have been added or dropped may include aligning the two sequences of the two alleles along the ROIs or repetitive segments to determine when a repeat motif is dropped or added. For example, the sequences may be aligned with each other at one end to determine when a repeat motif has been added or dropped.

Alternatively or in addition to the above, the analysis may include, at 173, comparing lengths of the repetitive segments of the first and second alleles to determine if the lengths of the repetitive segments between the first and second alleles differ by a length of one repeat motif of multiple repeat motifs. For example, in the example shown in Figure 9, the repeat motif is TAGA, which is a tetranucleotide having four nucleotides. The sequence lengths of the target alleles are shown in Figure 9. Each of Allele 1 and Allele 2 have 62 nucleotides, and each of Allele 3 and Allele 4 have 58 nucleotides. Accordingly, the sequence length of Allele 1 differs from the sequence of Allele 3 and the sequence of Allele 4 by four nucleotides or, in other words, a length of the repeat motif. Likewise, the sequence length of Allele 2 differs from the sequence of Allele 3 and the sequence of Allele 4 by a length of the repeat motif.

In some embodiments, the analysis, at 166, may include determining, at 174, whether the added or dropped repeat motif(s) is/are identical to an adjacent repeat motif in the same sequence. As described above, the added or dropped repeat motif(s) may be determined by aligning the allele sequences to identify the repeat motif(s) that have been added or dropped. After aligning the sequences, it may be determined that that repeat motif that was added/dropped was identical to the repeat motif adjacent to it. In some embodiments, alignment may be accomplished by using a greedy algorithm.

The first allele (or the allele suspected of being a stutter product) typically includes a read count (or count score) that is less than the read count (or count score) of the second allele. Under certain circumstances, such as when the sample includes a minor contributor, this may not be true. In some cases, the stutter product of an allele may be less than a designated stutter threshold or falls within a predetermined range for the locus and/or allele. The stutter threshold may be based on, for example, a number of read counts for the second allele, historical data of the corresponding locus and/or allele, and/or observations of the corresponding locus and/or allele during the assay. To provide an example regarding the historical data or observations of an allele, it may be determined through experience regarding a designated assay that an allele provides a predetermined amount of stutter that is greater than or less than generally expected. This data and/or observations may be used to modify the threshold. As another example in which the knowledge of an allele may affect the stutter threshold, longer alleles on average may provide a greater percentage of stutter product. Thus, the stutter threshold may be changed based on a length of the allele.

In some embodiments, the analysis, at 166, may include determining, at 175, whether the count scores of the first allele fall within a predetermined range of the count scores of the second allele. For example, if the count scores (e.g., read counts) of the first allele are within a predetermined percentile range of the count scores (e.g., read counts) of the second allele, then the first allele may be suspected stutter product. A predetermined percentile range may be between about 5% and about 40%. In particular embodiments, the predetermined percentile range may be between about 10% and about 30% or between about 10% and about 25%. The predetermined percentile range may be calculated or obtained using historical data or observations of the corresponding STR locus during the assay. Likewise, if the count scores of the first allele are less than a designated stutter threshold that is based on the count scores of the second allele, then the first allele may be suspected stutter product. By way of example, a designated stutter threshold may be based on a predetermined percentage of the count score of the second allele. For example, the predetermined percentage may be about 20%, 25%, 30%, 35%, or 40%. The predetermined percentage may be determined or obtained using historical data of the corresponding STR or observations of the corresponding STR locus during the assay.

In some embodiments, the count scores of a potential allele may be used to determine a stutter metric (or stutter score). The stutter metric may be a value or function that is based on count scores of the first allele. The stutter metric may also be based on the count scores of the second allele. The stutter metric may be compared to a designated stutter threshold to determine whether the corresponding potential allele is suspected stutter product. If the stutter metric is less than the designated stutter threshold, then the first allele may be suspected stutter product of the second allele. If the stutter metric is not less than the designated stutter threshold, then the first allele may be considered as a potential allele. In this case, the first allele and the second allele may each be true alleles of the locus.

Additional conditions may be applied to determine whether one allele is the stutter product of another allele. For example, the analysis, at 166, may include determining, at 176, that no other mismatches exist between the sequences of the first and second alleles. The ROIs or, more specifically, the repetitive segments may be analyzed to identify any mismatches between the respective sequences. For example, if a nucleotide of one sequence is not matched by the nucleotide of the other sequence (other than the added/dropped repeat motif(s)), then the sequences may not be stutter products.

In other embodiments, it may be determined that the suspected stutter product is not stutter product of a second allele. Instead, the suspected stutter product may be from another contributor or may be caused by sequencing error. For example, one or more embodiments may determine that the suspected stutter product is from another contributor if the stutter metric (e.g., the count score or other function based on the count score) of the first allele is greater than a designated stutter threshold. The designated threshold may be based on the count score for the second allele and a predetermined stutter function, which may be based on historical data and/or data within the assay-of-interest. One or more embodiments may determine that the suspected stutter product is sequencing error if the stutter metric of the first allele is less than a baseline value. The baseline value may be based on the count score for the second allele and a predetermined stutter function, which may be based on historical data and/or data within the assay-of-interest. By way of example, a certain locus may historically have a stutter range of 10-30%. If the read count of the second allele for the certain locus is 100, then the first allele may be sequencing error if the read count is less than 10. The first allele may possibly be from another contributor if the read count is greater than 30.

In particular embodiments, a first allele is considered to be the stutter product of a second allele, if: (A) the allele sequences of the first and second alleles differ in length by *k* repeat motifs; (B) the dropped or added repeat motif(s) are identical to the adjacent repeat motif; (C) there are no other mismatches between the two alleles (e.g., ROIs or repetitive segments); and, optionally, (D) the stutter metric of the first allele is within a predetermined stutter range (or less than a designated stutter threshold) of the stutter metric of the second allele.

Returning to the example shown in Figure 9, the sequences for the two true alleles of the D1S1656 locus are [TAGA]11[TAGG]1[TG]5 for allele 12 and [TAGA]13[TG]5 for allele 13. Allele 12 has a SNP in the last "TAGA" repeat unit. From this, we can determine that the allele 12 sequence [TAGA]12[TG]5 is in fact the -1 stutter of allele 13, and the allele 13 sequence [TAGA]12[TAGG]1[TG]5 is the +1 stutter of allele 12. As can be seen, embodiments set forth herein may be advantageous over CE systems. More specifically, CE systems would not be able to determine that the allele 12 sequence [TAGA]12[TG]5 is the -1 stutter of allele 13, and the allele 13 sequence [TAGA]12[TAGG]1[TG]5 is the +1 stutter of allele 12.

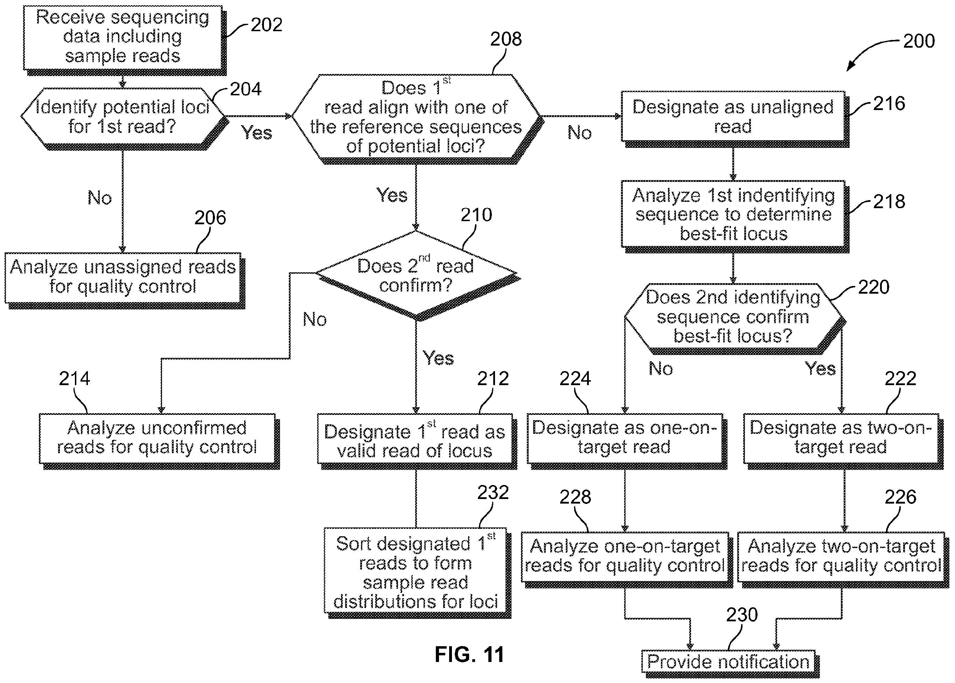

Figure 11 illustrates a method 200 of analyzing sequencing data in accordance with an embodiment. The method 200 may be incorporated with other embodiments set forth herein. The method 200 includes receiving, at 202, sequencing data including a plurality of sample reads that are configured to correspond to a set of genetic loci. The set of genetic loci may be configured for a predetermined genetic application, such as forensics or paternity testing. The sample reads may form read pairs of corresponding amplicons in which each read pair includes a first read and a second read of the corresponding amplicon. For example, the first and second read pairs may be obtained from pair-end sequencing and, in particular embodiments, asymmetric paired-end sequencing. Each of the first and second reads may have a respective sequence, hereinafter referred to as a read sequence. Each read sequence may include, for example, identifying sequences (e.g., primer sequences) and a sequence that includes a sequence variation, such as an SNP or STR.

The method 200 may include identifying, at 204, one or more potential genetic loci for the sample reads. The identifying operation may be similar to the assigning, at 154, described above with respect to Figure 2. For example, at 204, one or more genetic loci for a first read of a read pair may be provisionally identified. The first read of each read pair may be compared to select sequences of a database (e.g., look-up table). Each of the select sequences of the database may correspond to a designated genetic locus of the set of genetic loci. If the read sequence of the first read effectively matches one or more of the select sequences, then the first read may be provisionally called for the genetic loci that corresponds to the select sequences. For example, if a series of n nucleotides (e.g, the first n nucleotides) from an identifying sequence of the first read effectively matches one or more of the select sequences, then the first read may be provisionally called for those corresponding genetic loci. The corresponding genetic locus (or loci) may be referred to as the provisionally-designated locus (or loci).

If the first read does not effectively match with any of the select sequences, the unassigned read may be discarded. Optionally, the unassigned read, which may be the first read and/or the corresponding second read, may be collected or aggregated with other unassigned reads. At 206, the unassigned reads may be analyzed for quality control. For example, the read sequences of the first read may be analyzed to determine why the first read was not assigned.

The method 200 may also include determining, at 208, for each of the first reads that has a potential genetic locus, whether the first read aligns with one or more of the reference sequences of the potential genetic loci. The determination, a 208, may be made using one or more alignment protocols. For example, the determination, at 208, may include aligning the first reads to corresponding reference sequences of the potential genetic loci as described above with respect to Figures 3-7. If the first read aligns with the reference sequence of only one of the potential loci, then the first read may be provisionally-designated as a valid read of the one genetic locus and the method may proceed to step 210. In other embodiments, the first read may be designated as a valid read of the one genetic locus and the method may proceed to step 212.

However, if the first read effectively aligns with more than one reference sequence, the determining, at 208, may include identifying the reference sequence that the first read best aligns with or most aligns with. More specifically, although the first read may effectively align with multiple reference sequences, one alignment may be better than other alignments. As one simple example, an alignment analysis may analyze the first read and align the first read to three reference sequences, Ref Seq A, Ref Seq B, and Ref Seq C, which are the reference sequences that are associated with three potential genetic loci identified at 204. The alignment analysis may determine that the first read effectively aligns with Ref Seq A with a total of three differences between Ref Seq A and the first read. The alignment analysis may determine that the first read effectively aligns with Ref Seq B with a total of four differences between Ref Seq B and the first read. The alignment analysis may determine that the first read and Ref Seq C do not align with each other. For example, an excessive number of differences (e.g., above 10) may exist between the first read and Ref Seq C. As another example, an excessive proportion or percentage of differences (e.g., number of differences relative to a total number of nucleotides in the read or in the reference sequence) may exist between the first read and Ref Seq C. Based on this data, the method may determine that the first read aligns better with Ref Seq A than with Ref Seq B. Accordingly, the first read may be provisionally-designated as a valid read of the genetic locus that corresponds to Ref Seq A.

In some embodiments, determining which reference sequence aligns best with the first read may include computing alignment scores for each of the reference sequences, wherein the alignment scores are based on the number of differences. As described above, the alignment score may be a raw number (e.g., number of differences). In other embodiments, the alignment score may be a function of the number and/or types of differences. For instance, indels and mismatches may be scored differently.

Optionally, the method 200 may include analyzing, at 210, the second read to confirm that the first read should be called for the provisionally-designated genetic locus. The second read may be analyzed in a similar manner as the first read of the corresponding read pair. The second read may be analyzed to determine if an identifying sequence of the second read effectively matches one or more select sequences of a database. If the identifying sequence of the second read effectively matches only one select sequence, the method may include identifying the genetic locus that corresponds to the one select sequence. If the genetic locus is the same genetic locus that the first read was provisionally-designated to, then the genetic locus may be referred to as the genetic locus of the first read and the first read may be designated, at 212, as being a valid read of the genetic locus.

However, if the identifying sequence of the second read effectively matches multiple select sequences, the method may include identifying the genetic loci that correspond to the multiple select sequences. If one of these genetic loci is the same genetic locus that the first read was provisionally-designated to, then the genetic locus may be referred to as the genetic locus of the first read and the first read may be designated, at 212, as being a valid read of the genetic locus.

If analyzing, at 210, does not confirm that the second read corresponds to the provisionally-designated locus of the first read, then the method 200 may include designating the corresponding first read as an unconfirmed read. The unconfirmed reads may be collected and, optionally, further analyzed, at 214, for quality control. For example, read pairs that effectively matched with a first select sequence of the provisionally-designated locus, but did not effectively match with a second select sequence of the provisionally-designated locus may be indicative of concerns within the assay. The unconfirmed reads may indicate one or more off-target amplicons. The read pairs may be analyzed, at 214, to determine, for example, whether a quality-control issue exists with the assay or indicates allele drop-out.

However, if the first read does not align with a reference sequence of a potential genetic locus at 208, then the method may include designating, at 216, the first read as an unaligned lead. The unaligned reads may represent first reads that passed one filtering stage, but were not able to align with a reference sequence. In particular, the unaligned reads may be first reads that have been confirmed as having an identifying sequence that effectively matches with one or more select sequences, but were not able to align with a reference sequence.

Optionally, the method 200 may include analyzing, at 218, each of the unaligned reads to determine a best-fit genetic locus for the corresponding unaligned read. As described above, the identifying sequence may effectively match with more than one select sequence. The analyzing, at 218, may include comparing the identifying sequence of the unaligned read to the select sequences that were previously identified at 204. The best-fit genetic locus may be the genetic locus that corresponds to the select sequence that best matches or most matches with the identifying sequence of the unaligned read. Accordingly, at 218, the method may determine which select sequence, among the multiple select sequences, best matches with the identifying sequence. For example, the best-fit genetic locus may be the genetic locus that corresponds to the select sequence that has the fewest differences with the identifying sequence. In some embodiments, the analyzing, at 218, may include determining a matching score for each of the select sequences with respect to the identifying sequence. The genetic locus that corresponds to the select sequence with the greatest matching score may be designated as the best-fit genetic locus.

At 220, the second read associated with the unaligned read (i.e., the first read) may be analyzed to determine if the second read confirms the best-fit locus identified at 218. The second read may be analyzed to determine if the identifying sequence of the second read effectively matches with one or more select sequences. If the identifying sequence of the second read effectively matches with a select sequence and that select sequence corresponds to the best-fit genetic locus, then the unaligned read may be designated, at 222, as a two-on-target unaligned read (also referred to as a pair-on-target unaligned read). A two-on-target unaligned read may represent an unaligned read that has sequences proximate to both ends of the unaligned read that effectively match with select sequences from the database. Despite effectively matching with two select sequences, the ROI of the unaligned read was not able to align with a reference sequence.

However, if the identifying sequence of the second read does not effectively match with a select sequence that corresponds to the best-fit genetic locus, then the unaligned read may be designated, at 224, as a one-on-target unaligned read. A one-on-target unaligned read may represent an unaligned read having only one identifying sequence that effectively matches with a select sequence from the database.

Both the two-on-target unaligned reads and the one-on-target unaligned reads may be analyzed, at 226 and 228, respectively, for quality control purposes. The analysis, at 226 or 228, may include analyzing a total number of unaligned reads (or a comparable score) and/or analyzing the sequences of the ROI of the unaligned reads. For example, the one-on-target unaligned reads may be analyzed, at 228, to determine a health of the assay. More particularly, the one-on-target unaligned reads may be analyzed to determine if chimera exist and/or if primer dimers exist. An excessive number of chimera and/or primer dimers may indicate that the assay is poor (e.g., amplification issue) or that the sample DNA is of a low quality. Optionally, the analysis, at 228, may include analyzing the unconfirmed reads of 214 to determine a health of the assay. The analysis, at 228, may include collectively analyzing the unconfirmed reads and the one-on-target unaligned reads. Alternatively, the analysis, at 228, may include separately analyzing the unconfirmed reads and the one-on-target unaligned reads.

With respect to two-on-target unaligned reads, an excessive number of such reads may indicate a possible allele dropout. In some embodiments, the analysis, at 226, may include determining if the number of two-on-target unaligned reads exceeds a percentage of total reads for the designated locus, then it may be determined that an issue exists with the designated locus. The "total reads" of the designated locus may be a function of the valid reads, designated at 212, and the unaligned reads, designated at 216. For example, the total reads may be equal to the sum of the valid reads and the unaligned reads. In other embodiments, the total reads may also be a function of the unconfirmed reads. At 226, the number of two-on-target unaligned reads (or comparable score) may be compared to a threshold to determine whether an issue (e.g., allele dropout) exists with the designated locus.

At 230, a notification may be provided regarding the quality of the assay and/or the confidence in the genetic profile. For example, the notification may inform a user of a number of unaligned reads. In particular embodiments, the notification may inform the user of a number of one-on-target unaligned reads and/or a number of two-on-target unaligned reads. In some cases, the method may compare the number of unaligned reads (or comparable score), the number of one-on-target unaligned reads (or comparable score), and/or the number of two-on-target unaligned reads (or comparable score) to designated thresholds. If the numbers or scores exceed the thresholds, the notification may include a specific warning or specific guidance for the user. For instance, the notification may inform a user that evidence indicates that the sample was of poor quality and/or was in small amounts. The notification may be directed to the assay as a whole or may be specific to particular loci. More specifically, an excessive number of one-on-target unaligned reads may indicate a problem with the assay, whereas an excessive number of two-on-target unaligned reads may indicate allele dropout.

At232, the valid reads may be sorted to form a read distribution of the designated locus. The read distribution typically includes numerous sample reads that have been passed through multiple filtering stages and assigned to the designated locus. For example, the read distribution may include tens, hundreds, or thousands of first reads that have been assigned to the designated locus. The read distribution may be collected in a file (e.g., "distribution file") and include information regarding the distribution of the sample reads, such as different potential alleles, sequences of the alleles, and a count score (e.g., read count or other value/function based on the read count) for each potential allele. For example, when the valid reads are sorted for the read distribution, the valid reads may be sorted based on sequence. The valid reads may have a number of different sequences that, although different, have been assigned to the designated locus. Each different sequence represents a potential allele of the designated locus. One or more of the sequences may be noise (e.g., sequencing error), one or more of the sequences may be stutter product, and one or more of the sequences may be true alleles.

The valid reads may be aggregated with other valid reads that have the same sequence. The number of valid reads having the same sequence may be counted for the particular sequence. For instance, assuming a genetic locus having 1000 valid reads assigned thereto, the read distribution may indicate that eight different sequences exist. The valid reads may be distributed among the eight different sequences. For example, Allele 1 may have 10 valid reads; Allele 2 may have 20 valid reads, Allele 3 may have 10 valid reads; Allele 4 may have 400 valid reads; Allele 5 may have 15 valid reads; Allele 6 may have 500 valid reads; Allele 7 may have 25 valid reads; and Allele 8 may have 20 valid reads. Further analysis, as described below, may determine that some of the alleles are noise and/or stutter product.

In some embodiments, the potential alleles may be provided a CE Allele name that is based on conventional naming practices in CE. The CE Allele name for a potential allele may be based, in part, on the number of repeat motifs within the sequence. CE Allele naming may also be based historical usage. In some embodiments, the potential alleles are ordered within the read distribution based on the CE Allele name. For example, CE Allele names typically include a numerical value. The potential alleles may be ordered based on the numerical values. By way of one example, the graph 192 shown in Figure 10 illustrates one read distribution. As shown, the potential alleles include 11, 11.2, 12, 13, and 14. The read distribution for the genetic locus illustrated in graph 192 may be ordered 11, 11.2, 12, and 13.

Under some circumstances, two different potential alleles may have the same CE Allele name. For example, based on the conventional naming practices, the potential alleles may be given the same CE Allele name. In some embodiments, the read distribution may indicate that the two different sequences have the same CE Allele name. For example, the read distribution may indicate the CE Allele name (e.g., 13) and then list the different sequences that corresponded to the same CE Allele name.

After sorting the read to form read distributions, the read distributions may then be communicated for different analyses. For example, the genetic loci that are known for including SNPs may be directed through an SNP analysis. The genetic loci that are known for STRs may be directed through an STR analysis. Although the SNP and STR analyses may include different steps, the analyses may also include similar steps.

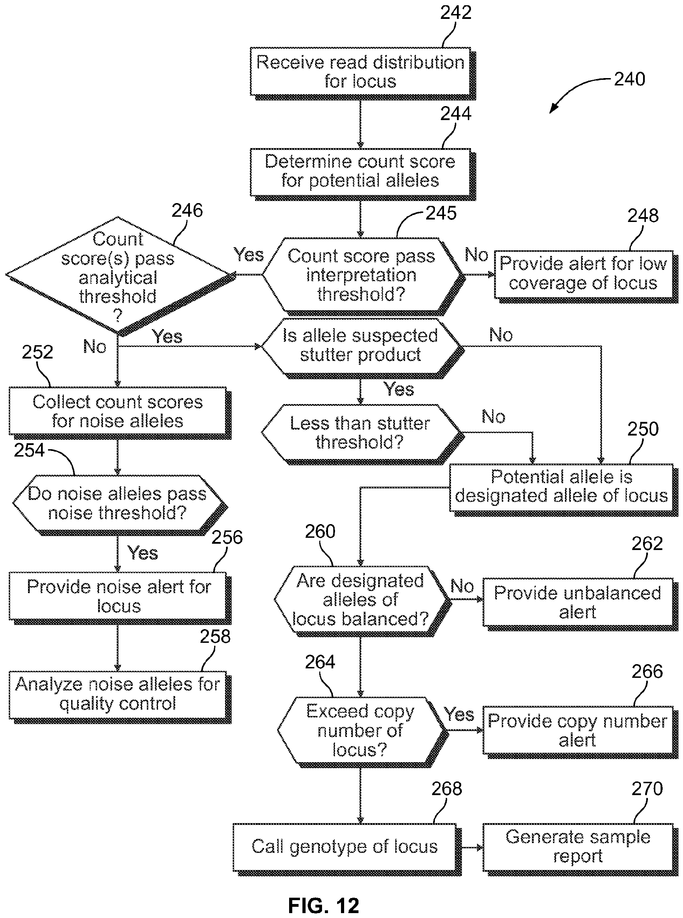

Figure 12 illustrates a method 240 of analyzing sequencing data in accordance with an embodiment. In particular, the method 240 includes analyzing read distributions of designated loci. The read distributions may be STR loci, SNP loci, or other loci associated with sequence variations. The method 240 includes receiving, at 242, read distributions for the designated loci. With respect to the following steps, each step may be at least partially based on the designated locus. For example, various functions (e.g., thresholds) may be applied in which those functions are based on the designated locus. More specifically, the function for one genetic locus may not be the same function of another genetic locus.

Optionally, the method 240 includes determining, at 244, a count score for each of the potential alleles for a designated genetic locus. The count score may be based on the read count of the potential allele. The read count represents the number of valid reads that include a common sequence. In some embodiments, the count score is a value that is equal to the read count for the potential allele. For example, if the read count is 300, then the count score may be 300. In other embodiments, the count score for a potential allele may be based on the read count and a total number of reads for the genetic locus. The total number of reads may be, for example, a total number of reads within the read distribution for all potential alleles. In some embodiments, the count score for a potential allele may be based on the read count and previously-obtained data of the genetic locus. In particular embodiments, the count scores may be normalized scores between predetermined numbers (e.g., 0 and 1). The normalized scores may be based on a total number of reads for the genetic locus. Optionally, the normalized scores are a function of the read counts from other loci and/or the read counts from other samples. The count score may also be a function of read counts from other loci of the sample or a function of read counts from other samples that were concurrently run with the sample-of-interest. The count score may also be a function of historical data. For example, different types of assays may be run to obtain read counts. In some embodiments, the count score is based on historical data regarding a particular assay.

The method 240 may also include determining, at 245, whether one or more of the count scores of the potential alleles passes an interpretation threshold. The interpretation threshold may be a predetermined value or may be a function that is based on a plurality of factors. For example, the interpretation threshold may be based on a number of total reads that correspond to the designated locus. The number of total reads may include the valid reads of all potential alleles within a locus. In some embodiments, the number of total reads may include the valid reads of the locus and the unaligned reads of the locus. In particular embodiments, the number of total reads may include the valid reads, the unaligned reads, and the unconfirmed reads of the locus. If one of the count scores passes the interpretation threshold at 245, then the method 240 may proceed to step 246 or another subsequent step. In some embodiments, the interpretation threshold may be based on a total number of reads in a sample. In some embodiments, the interpretation threshold may be based on a total number of reads in a plurality of samples.

If none of the count scores passes the interpretation threshold, at 245, then the method 240 may provide, at 248, an alert or other notification regarding the designated locus. For instance, the alert may inform a user that the designated locus has low coverage. More specifically, the alert may inform the user that the amount of data regarding the designated locus may be insufficient to provide a genotype call.

In a particular embodiment, the method 240 includes identifying the potential allele that has a maximum read count (or allele count) within the read distribution. The read count represents the number of valid reads that include a common sequence. With respect to STRs, the read count may represent the number of valid reads that include a common sequence of the ROI or the repetitive segment. The method 240 may also include comparing the maximum read count to an interpretation threshold. If the maximum read count passes the interpretation threshold, at 245, then the method 240 may proceed to step 246 or another subsequent step. If the maximum allele count does not pass the interpretation threshold, then the method 240 may provide, at 248, an alert or other notification regarding the designated locus as described above.

In other embodiments, the count score may be compared to another threshold, such as the analytical threshold described below. The analytical threshold is typically easier to pass than the interpretation threshold. If none of the potential alleles have a count score that passes the analytical threshold, then it may be determined that the genetic locus has low coverage. As another example for determining whether the genetic locus has enough coverage, a total number of reads for the genetic locus (e.g., valid reads) may be compared to a read threshold. The read threshold may be based on the total number of reads in the sample and/or historical data. If the total number of reads for the genetic locus does not pass the read threshold, then it may be determined that the genetic locus has low coverage. In other embodiments, a combination of one or more steps, such as those described above, may be used to determine whether the genetic locus has low coverage.

Optionally, at 246, each of the count scores or the corresponding read counts within the read distribution may be compared to an analytical threshold. Like the interpretation threshold, the analytical threshold may be a predetermined value or a function that is based on a plurality of factors, such as a total number of reads (e.g., total number of valid reads) for the locus and/or historical knowledge of the designated locus. The analytical threshold may be less stringent (e.g., easier to pass) than the interpretation threshold. More specifically, the interpretation threshold may require a larger read count to pass than the analytical threshold.

After passing the analytical threshold at 246, the method 240 may include determining, at 247, whether the potential allele is suspected stutter product. Various rules or conditions may be applied for determining whether the potential allele is suspected stutter product. For example, one or more of the factors 171-175 described above with respect to Figure 8 may be applied. In particular embodiments, the determining, at 247, includes determining whether a first allele has a repeat motif that has been added or dropped relative to a second allele.

If the potential allele is not suspected of being stutter product, the potential allele is designated, at 250, as being a designated or called allele of the locus. If the potential allele is suspected of being stutter product, the method 240 includes determining, at 249, whether the count score of the first allele is less than a designated threshold. The count score may be the read count or a function based on the read count. The designated threshold may be based on the count score of the second allele. In particular embodiments, the determination, at 249, may include determining whether the count score of the first allele is within a predetermined range (e.g., 10%-30%) of the count score of the second allele.

Although not indicated in Figure 12, if the potential allele is less than the designated threshold or within the predetermined range, the potential allele may be designated as stutter product of the second allele. The stutter product may be noted with the genotype call for the locus. For example, a sample report may include the genotype for the locus. with an indication that a stutter product exists. Information regarding the stutter product (e.g., sequence and percentage of second allele) may be provided within the sample report. However, if the count score or the read count passes a designated threshold (or is within the predetermined range), then the potential allele may be designated, at 250, as a designated allele of the genetic locus.

In some embodiments, the count scores of the noise alleles are collected, at 252. The noise alleles may include the potential alleles that did not pass the analytical threshold, at 246,. In some embodiments, the noise alleles may also include count scores from the unaligned reads and, optionally, the unconfirmed reads described above. The count scores for the noise alleles may be collected, at 252, and analyzed, at 254, to determine if an excessive number of reads indicate a potential issue with the corresponding locus. For example, the count scores of all the noise alleles may be summed and compared to a predetermined noise threshold. The noise threshold may be based on a total number of reads and/or historical data. If the noise threshold is passed, at 254, an alert may be provided, at 256, that the locus has an excessive amount of noise.

In some embodiments, the noise alleles may be analyzed, at 258, for quality control. In particular embodiments, the noise alleles for an STR locus may be sub-divided into noise alleles having sequences that are the same length as a called allele and noise alleles having sequences that are not the same length of the called alleles. Separation of the noise alleles may provide additional information as to why excessive noise exists with the corresponding locus.

After determining the designated alleles, at 250, the method 240 may include further analysis of the designated alleles before making a genotype call of the designated locus. A genotype call will typically include a heterozygous call (i.e., two different alleles) or a homozygous call (i.e., one observed allele). For heterozygous calls, the data will typically support that the reads are substantially evenly proportioned. If the two alleles are not represented substantially equal in the data, an issue may exist with the locus. Thus, in some embodiments, the method 240 may include analyzing, at 260, the called alleles to determine if the called alleles are balanced or in proportion. For example, a ratio of the called alleles may be calculated to determine if the ratio satisfies a balanced threshold. By way of example only, if the count score (e.g., read count) for one allele is less than 50% or less than 75% of the count score (e.g., read count) of another allele, the alleles may be designated as being unbalanced. Accordingly, an allele-proportion alert may be provided, at 262, indicating that the alleles are unbalanced. As discussed below, the allele-proportion alert may be analyzed with other evidence (e.g., other alerts) to determine whether the sample includes a plurality of sources.

In some embodiments, the method 240 may include determining, at 264, whether a copy number of the locus exceeds a copy threshold. For autosomal loci, the copy number will typically be at most two. For non-autosomal loci, such as X-loci or Y-loci, the copy number may be different. For example, the copy number of a Y-locus may be at most one. The copy number of a X-locus may be at most two. As described below, in some cases, a gender of the sample may be predicted and then used when querying whether a plurality of sources exist within the sample.

Accordingly, the determining, at 264, may include obtaining a copy number for the designated locus (e.g., 0, 1, or 2) and comparing the number of called alleles for the designated locus to the copy number. If the number of called alleles exceeds the copy number, an allele-number alert may be provided, at 266, that the locus includes an excessive number of alleles. As discussed below, the allele-number alert may be analyzed with other evidence (e.g., other alerts) to determine whether the sample includes a plurality of sources.

At 268, a genotype of the locus may be called. The genotype call is based on the designated alleles, at 250, and will typically be one or two alleles. However, in some embodiments, the genotype call will include more than two alleles. Genotype calls with more than two alleles may include notices that indicate an issue may be present at the locus or with the sample in general. At 270, a sample report may be generated that includes a genotype call, if possible, for the genetic loci of the predetermined set. The sample report may also include a number of notices (e.g., alerts) that have been identified by the method 240 or the method 200 (Figure 11). In some embodiments, a genotype call for a locus may be provided along with an indicator that notifies the reader of a potential issue (e.g., coverage, noise, allele dropout, stutter, etc.) regarding the locus. In other embodiments, a genotype call is not provided for a genetic locus if certain alerts for the genetic locus are identified (e.g., coverage or noise). In some embodiments, the sample report may include the sequences of the called alleles and, optionally, the sequences of stutter products and/or other identified potential alleles. In some embodiments, the sample report may include a confidence score with respect to the sample as a whole. For example, if a large number of one-on-target unaligned reads exist, the sample report may indicate that the sample may be of poor quality.

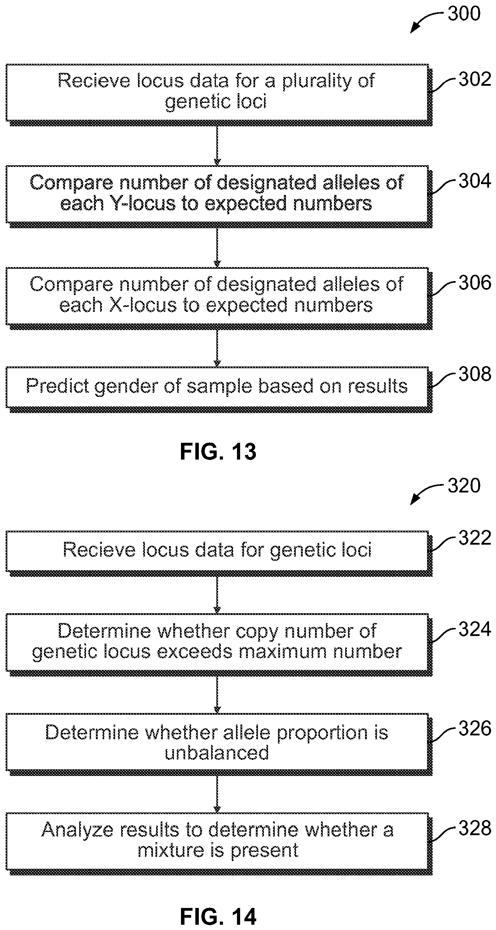

Figure 13 is a flowchart illustrating a method 300 of predicting a gender of the source of a sample. The method 300 assumes that the sample is from a single source. If it is subsequently determined that the sample is from multiple sources, as described below, then the gender prediction may be removed. In some embodiments, after determining that the sample includes multiple sources, the method may predict that all of the sources of the sample are a single gender, such as male.

The method 300 may be incorporated with the method 240 (Figure 12). The method 300 may be executed after determining the designated alleles for each genetic locus from a set of genetic loci. For example, the method 300 may be executed after step 250 in Figure 12 has occurred for all potential alleles for a plurality of genetic loci within a set of genetic loci (or for all genetic loci within the set). The method 300 includes receiving, at 302, locus data for a plurality of genetic loci. The locus data may include one or more designated (or called) alleles for the corresponding genetic loci. The plurality of genetic loci may be loci that are expected to have different numbers of alleles based on the gender of the sample. In other words, the locus data may correspond to the X- and Y-loci. The X-loci may include known SNP or STR loci on the X-chromosome. The Y-loci may include known SNP or STR loci on the Y-chromosome.

The method 300 may include comparing, at 304, a number of designated alleles of each Y-locus to an expected number if the sample is a male and/or to an expected number if the sample is female. The expected number may be a pre-set number based on historical data. The expected number of designated alleles for a male sample may be based on the number of times the locus or alleles appears on the Y-chromosome. Although this is typically one, it may be more than one (e.g., two). The expected number of designated alleles for a female sample within a Y-locus is zero.

Optionally, the method 300 may include comparing, at 306, a number of designated alleles of each X-locus to an expected number if the sample is a male and/or to an expected number if the sample is female. The expected number of designated alleles for a male sample within a X-locus is typically one, but may be more than one if the locus or allele appears more than one time on the X-chromosome. The expected number of designated alleles for a female sample within a X-locus is typically two, but may be more if the locus/allele appears more than one time on the X-chromosome.

The method 300 also includes predicting, at 308, a gender of the sample based on the results from the comparing, at 304, and/or the results from the comparing, at 306. Ideally, each of the Y-loci would include one designated allele if the sample was male and would include zero designated alleles if the sample was female. Likewise, each of the X-loci would ideally include one designated allele if the sample was male and would include one or two designated alleles if the sample was female. However, due to sequencing error, contamination, improper analysis, etc., it is possible that the X-loci and Y-loci would not be consistent in predicting a gender of the sample. In some cases, the analysis may consider numerous genetic loci. For example, there may be about five (5) to ten (10) Y-loci and about twenty (20) to thirty (30) X-loci. Thus, although the sample may be male, it is possible that one or more of the Y-loci may have zero designated alleles. Likewise, although the sample may be female, it is possible that one or more of the Y-loci may have a designated allele.

Accordingly, the analysis for predicting the gender of the sample may include analyzing a totality of the evidence to predict a gender of the sample. For instance, the analysis may include counting at least one of (i) a number of Y-loci that are consistent with the sample being a male; (ii) a number of Y-loci that are consistent with the sample being a female; (iii) a number of X-loci that are consistent with the sample being a male; (iv) or a number of X-loci that are consistent with the sample being a male. In some embodiments, only the numbers for the Y-loci may be considered in the analysis, at 308, or, alternatively, only the numbers for the X-loci may be considered. In some embodiments, the numbers for both the X- and Y-loci may be considered in the analysis, at 308. In some embodiments, one or more of the X-loci and/or one or more of the Y-loci may be given greater weight than other loci.

By way of one example, the analysis may review ten Y-loci. If nine of the ten Y-loci include a designate allele, which are consistent with the sample being male, the gender of the sample may be predicted to be male. If one of the ten Y-loci includes a designated allele, the gender of the sample may be predicted to be female. In some embodiments, the analysis may determine that the sample includes a mixture. For example, if the analysis, at 308, determines the number of Y-loci and the number of X-loci support both male and female samples, a mixture of sources may be predicted.

Figure 14 is a flowchart illustrating a method 320 of detecting whether a sample includes a mixture of sources. The method 320 may be incorporated with the method 240 (Figure 12) and, optionally, may be performed after predicting a gender of the sample. The method 300 includes receiving, at 322, locus data for each genetic locus of a set of genetic loci. The locus data may include one or more designated or called alleles for the corresponding genetic locus. The locus data may also include count scores (e.g., read counts) for the designated alleles, count scores for noise alleles, and count scores for stutter product. The count scores may be obtained as described herein.

For each genetic locus, the method 320 may include determining, at 324, whether a copy number of the genetic locus exceeds a maximum allowable number of alleles (herein after referred to as "maximum allele number"). As described above, the maximum allele number for autosomal loci is typically two. The maximum allele number for X-loci or Y-loci is based on whether the sample (assuming single source sample) is male or female. If the sample is male, the maximum allele number of a Y-locus is one and the maximum allele number for an X-locus is one. If the sample is female, the maximum allele number of a Y-locus is zero and the maximum allele number of an X-locus is two. The sample may be predicted to be male or predicted to be female based on the method 300 described above.

Accordingly, the determining, at 324, may include obtaining the maximum allele numbers for the genetic loci (e.g., 0, 1, 2) and comparing the copy number (i.e., the number of designated alleles) for each of the genetic loci to the corresponding maximum allele number. If the copy number exceeds the maximum allele number, an allele-number alert or flag may be provided for the genetic locus.

For each genetic locus, the method 300 may also include determining, at 326, whether an allele proportion of the designated alleles is unbalanced. As described above, the allele proportion of a genetic locus may be based on a count score (e.g., read count) for a first designated allele and a count score (e.g., read count) for a second designated allele. It may be expected that a single source sample be homozygous at a genetic locus or heterozygous at a genetic locus. If heterozygous, it may be expected that the allele proportion would be about a 1:1 ratio. A substantially disproportionate ratio may be indicative of the genetic locus not being heterozygous or the sample including more than one source. More specifically, the greater the calculated ratio deviates from 1:1, the greater the likelihood that the genetic locus is either homozygous or the sample, as a whole, includes a mixture of sources. As described below, determining that the sample includes a mixture of sources is based on analyzing multiple genetic loci (e.g. all genetic loci that were called).

In some embodiments, the determining, at 326, may include calculating a balance score that is based on a ratio of the count scores between the two designated alleles of the genetic locus. If the balance score is not within a designated range, such as 0.8:1.0 to about 1.2:1.0, the balance score may indicate that the allele proportion is unbalanced. If the genetic locus is determined to have an unbalanced allele proportion, an allele-proportion alert may be generated for the genetic locus. In some embodiments, the balance score may be compared to a designated threshold to determine whether the allele proportion is unbalanced.

The method 320 may also include analyzing, at 328, the results of the determination, at 324, and the determination, at 326, to determine whether a plurality of sources exists within the sample. The analysis, at 328, may be based on a number of allele-number alert(s) and a number of allele-proportion alerts(s) for the set of genetic loci. In one embodiment, a total number of the alerts may be calculated. If the total number of alerts exceeds a mixture threshold, then the sample may be flagged for having a plurality of sources. The mixture threshold may be based on the number of genetic loci that were analyzed (i.e., the number of genetic loci in the set of genetic loci). In particular embodiments, the mixture threshold may be based on the number of genetic loci that were called. In some embodiments, the mixture threshold is based on historical data or knowledge with respect to a particular assay.

In some embodiments, the set of genetic loci may include, for example, 10, 20, 30, 40, 50, 60, 70, 80, 90, 100 genetic loci or more. In particular embodiments, the set of genetic loci may include 120, 140, 160, 180, 200 genetic loci or more. In more particular embodiments, the set of genetic loci may include 250, 300, 350 genetic loci or more.

In some embodiments, the mixture threshold is a predetermined value that is equal to a predetermined percentage of the genetic loci within the set. The predetermined percentage may be at least, for example, 5%, 10%, 15%, 20%, 25%, 30%, 35%, 40%, 50%, 60%, 70%, or more.

In some embodiments, the allele-number alert may include an allele-number score that is based on the number of designated alleles. More specifically, a likelihood of the sample including a mixture may increase as the number of designated alleles beyond the maximum number of allowable alleles for the genetic locus increases. To illustrate, if the number of designated alleles for a first genetic locus was three (3) and the number of designated alleles for a second genetic locus was (4), the allele-number score for the second genetic locus may be assigned a greater value (or a greater weight) than the allele-number score for the first genetic locus when determining whether a mixture exists.

In some embodiments, the allele-proportion alert may include an allele- proportion score that is based on the proportion of the designated alleles of a genetic locus. More specifically, a likelihood of the sample including a mixture may increase as the proportion of designated alleles becomes more disproportionate. For example, if the allele proportion for a first genetic locus was 1.3:1.0 and the allele proportion for a second genetic locus was 2.0:1.0, the allele-number score for the second genetic locus may be assigned a greater value (or a greater weight) than the allele-proportion score for the first genetic locus when determining whether a mixture exists.

In some embodiments, the sample report may include a mixture alert that informs the user that the sample is suspected of containing a plurality of sources. In some embodiments, the mixture alert may be accompanied by a confidence score that informs the user a level of confidence in the mixture alert. The confidence score may be based on at least one of a number of allele-number alerts, the allele-number scores associated with the allele-number alerts, a number of allele-proportion alerts, and the allele-proportion score associated with the allele-proportion alerts.

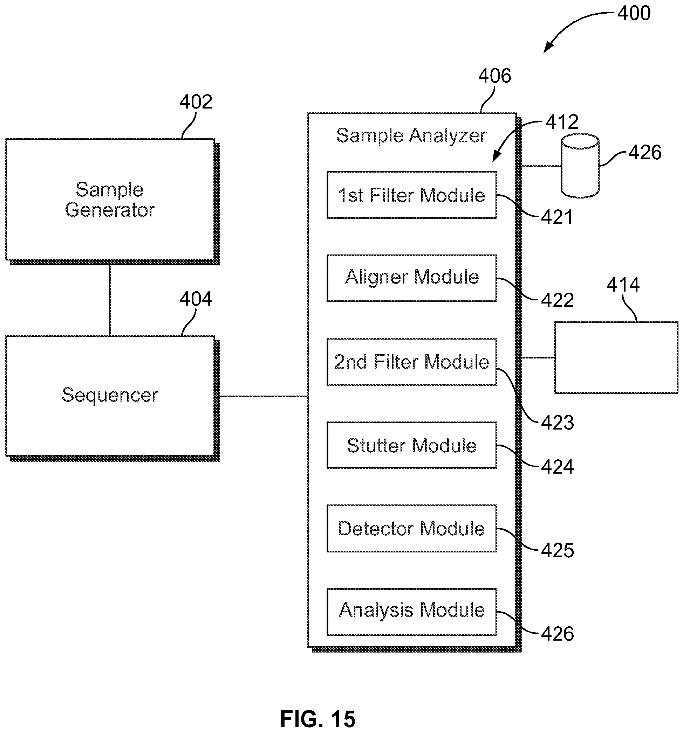

Figure 15 illustrates a system 400 formed in accordance with an embodiment that may be used to carry out various methods set forth herein. For example, the system 400 may be used to carry out one or more of the methods 100 (Figure 1), 150 (Figure 1), 160 (Figure 8), 200 (Figure 11), 240 (Figure 12), 300 (Figure 13), and 340 (Figure 14). Various steps may be automated by the system 400, such as sequencing, whereas one or more steps may be performed manually or otherwise require user interaction. In particular embodiments, the user may provide a sample (e.g., blood, saliva, hair semen, etc.) and the system 400 may automatically prepare, sequence, and analyze the sample and provide a genetic profile of the source(s) of the sample. In some embodiments, the system 400 is an integrated standalone system that is located at one site. In other embodiments, one or more components of the system are located remotely with respect to each other.

As shown, the system 400 includes a sample generator 402, a sequencer 404, and a sample analyzer 406. The sample generator 402 may prepare the sample for a designated sequencing protocol. For example, the sample generator may prepare the sample for SBS. The sequencer 404 may conduct the sequencing to generate the sequencing data. As described above, the sequencing data may include a plurality of sample reads. Each sample read may include a sample sequence. In particular embodiments, the sample reads form read pairs that are generated from paired-end sequencing or, more particularly, asymmetric paired-end sequencing.

The sample analyzer 406 may receive the sequencing data from the sequencer 404. Figure 15 includes a block diagram of a sample analyzer 406 formed in accordance with one embodiment. The sample analyzer 406 may be used to, for example, analyze sequencing data to provide a genotype call for a particular locus or generate a genetic profile of a sample. The sample analyzer 406 includes a system controller 412 and a user interface 414. The system controller 412 is communicatively coupled to the user interface 414 and may also be communicatively coupled to the sequencer 404 and/or the sample generator 402.

In an exemplary embodiment, the system controller 412 includes one or more processors/modules configured to process and, optionally, analyze sequencing data in accordance with one or more methods set forth herein. For instance, the system controller 412 may include one or more modules configured to execute a set of instructions that are stored in one or more storage elements (e.g., instructions stored on a tangible and/or non-transitory computer readable storage medium, excluding signals) to process the sequencing data. The set of instructions may include various commands that instruct the system controller 412 as a processing machine to perform specific operations such as the workflows, processes, and methods described herein. By way of example, the sample analyzer 406 may be or include a desktop computer, laptop, notebook, tablet computer, or smart phone. The user interface 414 may include hardware, firmware, software, or a combination thereof that enables an individual (e.g., a user) to directly or indirectly control operation of the system controller 412 and the various components thereof. As shown, the user interface 414 includes an operator display 410.

In the illustrated embodiment, the system controller 412 includes a plurality of modules or sub-modules that control operation of the system controller 412. For example, the system controller 412 may include modules 421-426 and a storage system 426 that communicates with at least some of the modules 421-426. The modules include a first filter module 421, an aligner module 422, a second filter module 423, a stutter module 424, a detector module 425, and an analysis module 426. The system 400 may include other modules or sub-modules of the modules that are configured to perform the operations described herein. The first filter module 421 is configured to analyze sample reads to determine whether the sample reads are confirmed reads of a designated locus as set forth herein. The aligner module 422 is configured to analyze the confirmed reads and determine whether the confirmed reads are aligned reads of the designated locus as set forth herein. The second filter module 423 is configured to receive the valid reads and determine whether the valid reads represent potential alleles of the corresponding locus as set forth herein. The stutter module 424 is configured to determine whether a valid read is stutter product of another allele as set forth herein. The detector module 425 is configured to determine whether any errors or alerts should be indicated for corresponding loci as set forth herein. For example, the detector module 425 may determine that a locus has an excessive number of unaligned reads, low coverage, an excessive number of noise alleles, alleles that are unbalanced, and/or a mixture of alleles from different sources. The analysis module 426 is configured to determine a genotype for the genetic loci as described herein.

As used herein, the terms "module", "system," or "system controller" may include a hardware and/or software system and circuitry that operates to perform one or more functions. For example, a module, system, or system controller may include a computer processor, controller, or other logic-based device that performs operations based on instructions stored on a tangible and non-transitory computer readable storage medium, such as a computer memory. Alternatively, a module, system, or system controller may include a hard-wired device that performs operations based on hard-wired logic and circuitry. The module, system, or system controller shown in the attached figures may represent the hardware and circuitry that operates based on software or hardwired instructions, the software that directs hardware to perform the operations, or a combination thereof. The module, system, or system controller can include or represent hardware circuits or circuitry that include and/or are connected with one or more processors, such as one or computer microprocessors.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

In some embodiments, a processing unit, processor, module, or computing system that is "configured to" perform a task or operation may be understood as being particularly structured to perform the task or operation (e.g., having one or more programs or instructions stored thereon or used in conjunction therewith tailored or intended to perform the task or operation, and/or having an arrangement of processing circuitry tailored or intended to perform the task or operation). For the purposes of clarity and the avoidance of doubt, a general purpose computer (which may become "configured to" perform the task or operation if appropriately programmed) is not "configured to" perform a task or operation unless or until specifically programmed or structurally modified to perform the task or operation.

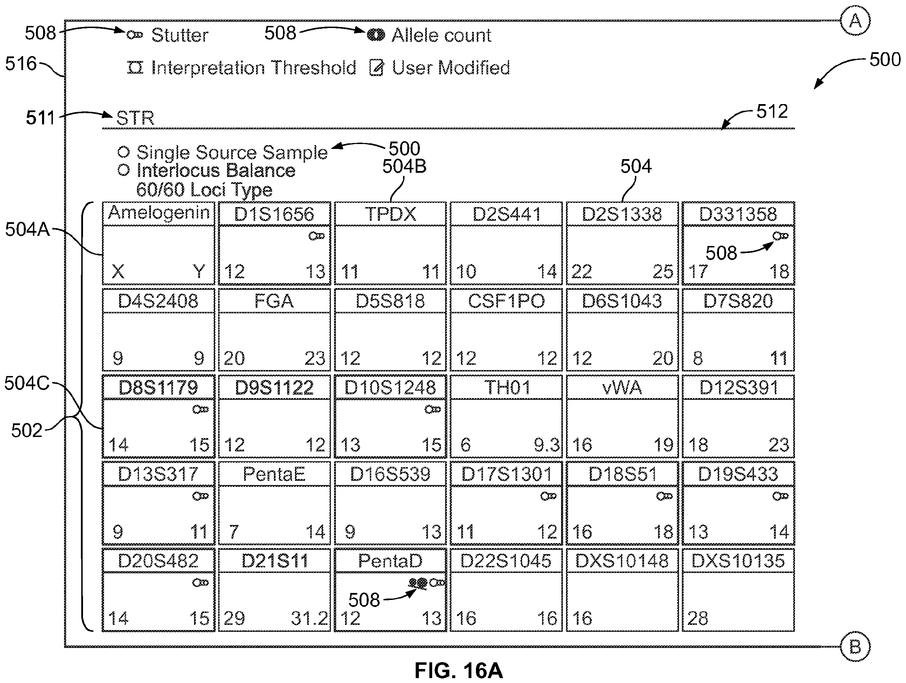

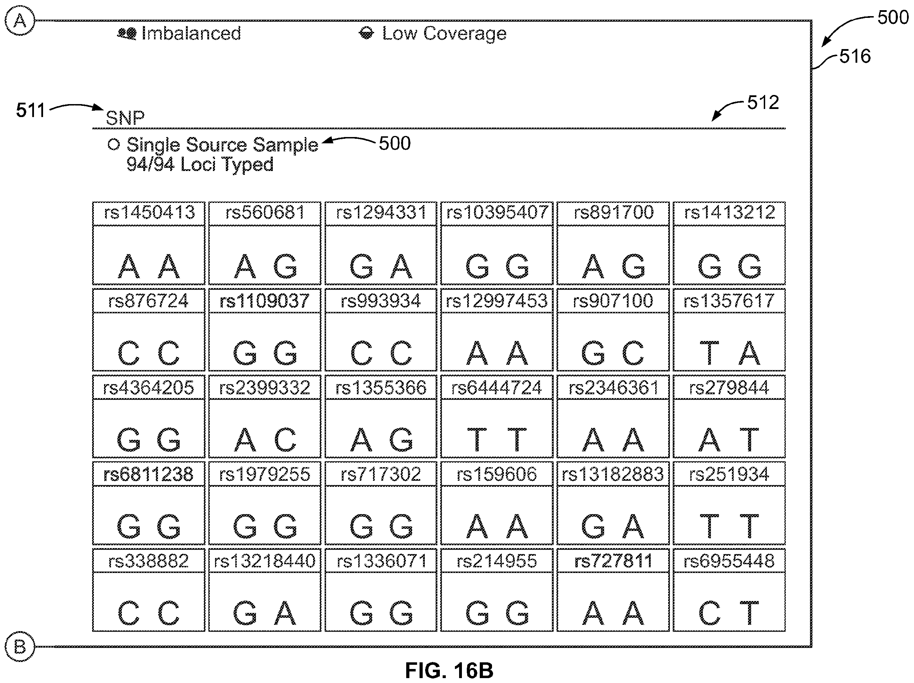

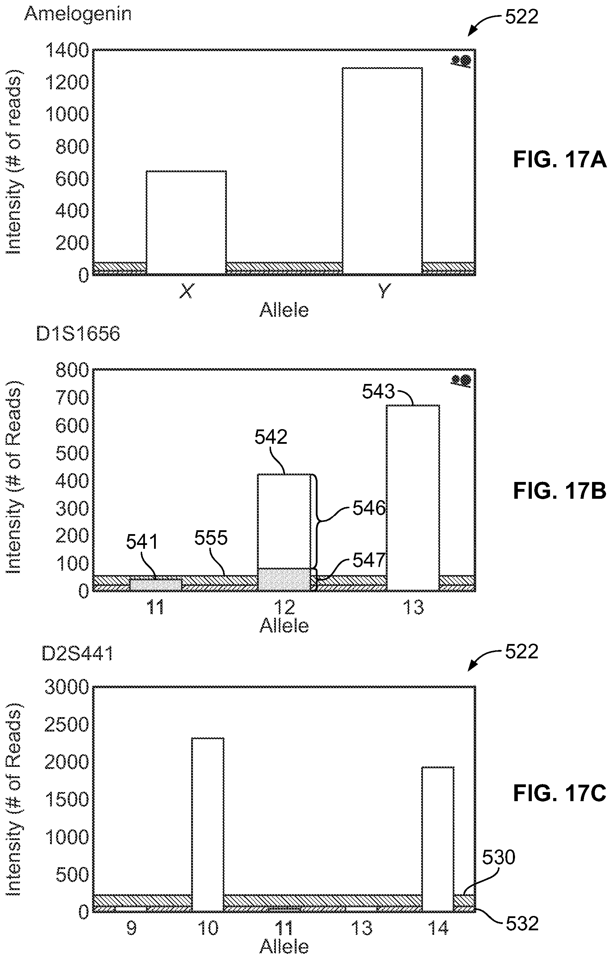

Figures 16A, 16B and 17A-17F illustrate examples of sample reports 500, 520 that may be generated by embodiments described herein. The sample reports 500, 520 may be stored in one or more files and transmitted through a communication network. The sample reports 500, 520 may be, for example, displayed on a screen or printed on paper. Figures 16A and 1613 illustrates only a portion of the entire sample report 500. As shown, the sample report 500 may include an overview or summary analysis of what is initially believe to be a single source sample. The sample report 500 includes a first section 511 for STR analysis and a second section 512 for SNP analysis. The sample report 500 may confirm that the sample is single source with a flag or indicator 510.

The sample report 500 includes an array 502 of call boxes 504. Each call box 504 may correlate to a designated genetic locus. For example, the call box 504A corresponds to the genetic locus Amelogenin, and the call box 504B corresponds to the genetic locus TPOX. Each of the call boxes 504 includes a genotype call 506 for the genetic locus. The genotype call 506 for Amelogenin is X, Y, and the genotype call for TPOX is alleles 11, 11. The names of the alleles may be based on conventional naming or may be determined through other naming protocols (e.g., proprietary protocol).

Each of the call boxes 504 may indicate whether a flag or notice is associated with the genetic locus. For example, in Figure 16, the call boxes 504 are color-coded to indicate whether a flag or notice exists. The call box 504A is gray, and the call box 504C is orange or red. Other methods of indicating may be used in alternative embodiments. In Figure 16, each of the call boxes 504 that is color-coded includes a flag 508. The flags 508 are referenced above in a legend 516 that defines the flags 508. For example, the sample report 500 includes flags 508 for "stutter," "allele count," "imbalanced," "low coverage," "interpretation threshold," and "user modified." These flags 508 may be assigned to the call boxes 504 after, for example, the analysis described herein.

Figures 17A-17F provides a more detailed analysis of the genetic loci. In some embodiments, the sample report 520 may be part of the sample report 500 (Figure 16). As shown, each of the genetic loci is assigned a graph 522 that visually represents the data for the corresponding genetic locus. In the illustrated embodiment, the graph 522 is a bar chart, but other graphs may be used to visually represent the data. The graph 522 specifically illustrates a read intensity relative to the different alleles. The read intensity may be the count score or based on the count score as described above. In some embodiments, the read intensity/count score is the read count.

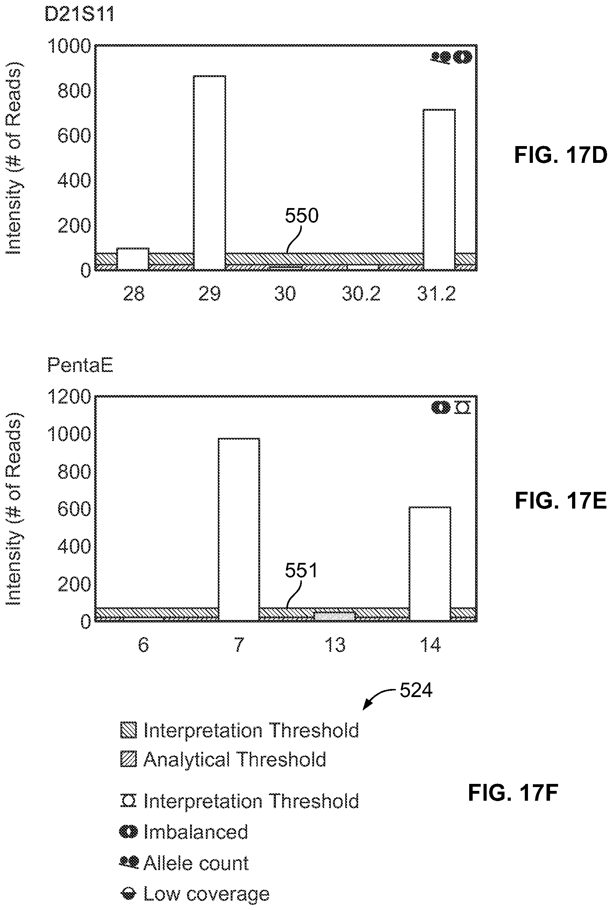

The graphs 522 may indicate an interpretation threshold and an analytical threshold with respect to the read intensity (or count score). For example, the D2S441 locus has an interpretation threshold 530 and an analytical threshold 532. The interpretation and analytical thresholds 530, 532 may be similar to the interpretation and analytical thresholds described above. As shown in Figure 17, the interpretation and analytical thresholds may be different for different loci. For example, the D21 S11 locus has an interpretation threshold 550 that is greater than an interpretation threshold 551 of the PentaE locus. As described above, the interpretation threshold and/or the analytical threshold may be based on (i.e., a function of) a total number of reads that correspond to the designated locus. Optionally, the interpretation thresholds and/or the analytical thresholds may be a function of the read counts for the particular locus and also a function of read counts from other loci and/or read counts from other samples.

In some embodiments, the graphs 522 may also indicate stutter product. The graphs 522 may visually differentiate the stutter product from true alleles. For example, the D1S1656 locus includes bars 541-543 that correlate to the CE Alleles 11, 12, and 13, respectively, of the D1S1656. The bars 541-543 may indicate a read intensity (or count score) of the respective alleles. The alleles of the D1S1656 locus shown in Figure 17 have been historically based on CE data and have been labeled, by convention, 11, 12, and 13. As indicated by different colors (e.g., blue and brown) in Figure 17, the alleles of the D1S1656 locus may include stutter product. More specifically, the bar 541 is stutter product and does not exceed the interpretation threshold 555 of the D1S1656 locus. The bar 542 includes bar portions 546, 547. Each of the bar portions 546, 547 visually represents a read intensity. Although the reads that correspond to the bar portions 546, 547 have the same sequence length, the reads that correspond to the bar portions 546, 547 have different sequences. The bar portion 546 represents stutter product. However, as described above, the stutter product represented by the bar portion 546 may be of another allele, such as the CE Allele 13. Accordingly, the color coding (or other indicator that differentiates the stutter product and true alleles) may notify or alert the user to analyze the different sequences of the CE alleles 11, 12, 13 to provide a more confident determination of the genetic call. In Figure 17, the genetic call of the D1S1656 locus is 12/13. In other cases, however, analyzing the sequences of the stutter product may change the genetic call. More specifically, in some cases, the genetic call using known CE processes would be incorrect. Embodiments set forth herein may be capable of providing the correct genetic call.

The sample report 520 also provides flags or notices for the different genetic loci. A legend 524 defines the notices. By way of one example, the D21S11 locus has flags for "imbalanced" and "allele count." In other words, the sample report 520 indicates to the viewer that the number of alleles is not expected and that the balance of the alleles is not expected. The viewer may wish to further investigate the data regarding the D21S11 locus.

In an embodiment, a method is provided. The method includes receiving sequencing data that includes a plurality of sample reads that have corresponding sequences of nucleotides. The method also includes assigning the sample reads to designated loci based on the sequence of the nucleotides, wherein the sample reads that are assigned to a corresponding designated locus are assigned reads of the corresponding designated locus. The method also includes analyzing the assigned reads for each designated locus to identify corresponding regions-of-interest (ROls) within the assigned reads. Each of the ROIs have one or more series of repeat motifs in which each repeat motif of a corresponding series includes an identical set of the nucleotides. The method also includes sorting, for designated loci having multiple assigned reads, the assigned reads based on the sequences of the ROIs such that the ROIs with different sequences are assigned as different potential alleles. Each potential allele has a sequence that is different from the sequences of other potential alleles within the designated locus. The method also includes analyzing, for designated loci having multiple potential alleles, the sequences of the potential alleles to determine whether a first allele of the potential alleles is suspected stutter product of a second allele of the potential alleles. The first allele is the suspected stutter product of the second allele if *k* repeat motifs within the corresponding sequences have been added or dropped between the first and second alleles, wherein *k* is a whole number. Optionally, *k* is equal to 1 or 2.

In one aspect, analyzing, for the designated loci having multiple potential alleles, the sequences of the potential alleles to determine whether the first allele is the suspected stutter product of the second allele may include comparing lengths of the ROIs of the first and second alleles to determine if the lengths of the ROIs of the first and second alleles differ by one repeat motif or multiple repeat motifs.

In another aspect, analyzing, for the designated loci having multiple potential alleles, the sequences of the potential alleles to determine whether the first allele is the suspected stutter product of the second allele may include identifying the repeat motif(s) that have been added or dropped and determining whether the added or dropped repeat motif(s) is/are identical to an adjacent repeat motif in the corresponding sequences.

In another aspect, the first allele may be the stutter product of the second allele if no other mismatches exist between the sequences of the ROIs of the first and second alleles.

In another aspect, the method may also include generating a genotype profile, the genotype profile calling a genotype for at least a plurality of the designated loci, wherein the designated loci having suspected stutter product are indicated as having the suspected stutter product.

In another aspect, the method may also include providing genotype calls for at least a plurality of the designated loci, wherein at least one of the genotype calls indicates that suspected stutter product exists for the designated locus of the at least one genotype call.

In another aspect, the method may also include counting, for each designated locus having multiple potential alleles, a total number of the sample reads called for the potential allele. The first allele may be the stutter product of the second allele if the sample reads of the first allele are less than a designated threshold of the sample reads of the second allele. Optionally, the designated threshold is about 40% of the sample reads of the second allele. Optionally, the suspected stutter product is designated as from another contributor if the sample reads of the first allele exceed a predetermined percentage of the sample reads of the second allele. Optionally, the suspected stutter product is designated as noise if the sample reads of the first allele are less than a predetermined percentage of the sample reads of the second allele.

In another aspect, the assigned reads include first and second conserved flanking regions having a corresponding repetitive segment located therebetween. For each assigned read, the method may include (a) providing a reference sequence comprising the first conserved flanking region and the second conserved flanking region; (b) aligning a portion of the first flanking region of the reference sequence to the corresponding assigned read; (c) aligning a portion of the second flanking region of the reference sequence to the corresponding assigned read; and (d) determining the length and/or the sequence of the repetitive segment.

Optionally, the aligning a portion of the flanking region in one or both of steps (b) and (c) includes: (i) determining a location of the corresponding conserved flanking region on the assigned read by using exact k-mer matching of a seeding region which overlaps or is adjacent to the repetitive segment and (ii) aligning the flanking region to the assigned read.

Optionally, the seeding region includes a high-complexity region of the conserved flanking region. For example, the high-complexity region may include a sequence that is sufficiently distinct from the repetitive segment so as to avoid mis-alignment. As another example, the high-complexity region may include a sequence having a diverse mixture of nucleotides.

Optionally, the seeding region avoids low-complexity regions of the corresponding conserved flanking region. For example, the low-complexity regions may have sequences that substantially resemble a plurality of the repeat motifs.

Optionally, the seeding region is directly adjacent to the repetitive segment; the seeding region may include a portion of the repetitive segment; or the seeding region is offset from the repetitive segment.

In another aspect, the sample reads may be PCR amplicons having forward and reverse primer sequences.

In another aspect, assigning the sample reads to the designated loci may include identifying sequences of the sample reads that correspond to primer sequences of PCR amplicons.

In another aspect, the sequencing data may be from a sequencing-by-synthesis (SBS) assay.

In another aspect, the ROI includes a short tandem repeat (STR). Optionally, the STR is selected from at least one of the CODIS autosomal STR loci, the CODIS Y-STR loci, the EU autosomal STR loci, or the EU Y-STR loci.

In an embodiment, a method is provided that includes receiving sequencing data having a plurality of sample reads of amplicons that correspond to a set of genetic loci. The sample reads include read pairs in which each read pair of a corresponding amplicon includes first and second reads of the corresponding amplicon. Each of the first and second reads has a respective read sequence. The method also includes identifying potential genetic loci for the first reads based on analysis of the read sequences of the first reads. The potential genetic loci are from the set of genetic loci. The method also includes determining, for each of the first reads having at least one potential locus, whether the first read aligns with a reference sequence of each of the potential genetic loci. If the first read aligns with a reference sequence of only one genetic locus, the method includes determining that the first read includes a potential allele of the one genetic locus. If the first read aligns with more than one reference sequence, the method includes determining that the first read includes a potential allele of the genetic locus having the reference sequence that best aligns with the first read. If the first read does not align with a reference sequence, the method includes designating the first read as an unaligned read and analyzing the unaligned read to identify a genetic locus from the potential genetic loci that best fits with the unaligned read. The method also includes generating a genetic profile that includes called genotypes for at least a plurality of the genetic loci, wherein the called genotypes are based on the potential alleles of the corresponding genetic loci. The genetic profile also includes one or more notifications for genetic loci having unaligned reads.

In one aspect, at least one of the notifications includes a confidence score associated with the corresponding genetic locus. The confidence score may be based on a number of unaligned reads that best fit with the corresponding genetic locus, wherein a greater number of unaligned reads indicates that the called genotype is less trustworthy.

In another aspect, analyzing the unaligned read to identify a genetic locus from the potential genetic loci that best fits with the unaligned read may include analyzing an identifying sub-sequence of the unaligned read to identify the genetic locus that best fits with the identifying sub-sequence.

In another aspect, the identifying sub-sequence includes at least a portion of a primer sequence.

In another aspect, identifying potential genetic loci for the first reads includes determining that primer sequences of the first reads effectively match sequences associated with the potential genetic loci.

In another aspect, the sequencing data is generated through asymmetric paired-end sequencing.

In another aspect, the method may also include analyzing the unaligned reads to determine whether a potential allele dropout exists.

In another aspect, the method may also include analyzing the unaligned reads to determine a health of the assay.

In another aspect, the method may also include analyzing the unaligned reads to determine whether the unaligned reads are indicative of a chimera.

In another aspect, the method may also include analyzing the unaligned reads to determine a number of primer dimers.

In another aspect, determining that the first read includes a potential allele of the genetic locus may include confirming that the second read corresponding to the first read also correlates to the genetic locus.

In another aspect, the method may also include analyzing the unaligned reads to determine if the unaligned reads are one-on-target reads or pair-on-target reads. The pair-on-target reads may have first and second identifying sub-sequences that effectively match with first and second select sequences of a database. The one-on-target reads may have only the first identifying sub-sequence effectively matching the first select sequence of a database.

In an embodiment, a method is provided that includes receiving sequencing data having a plurality of sample reads of amplicons that correspond to a set of genetic loci. The sample reads include read pairs in which each read pair of a corresponding amplicon includes first and second reads of the corresponding amplicon. Each of the first and second reads has a respective read sequence. The method also includes identifying potential genetic loci for the first reads based on analysis of the read sequences of the first reads. The potential genetic loci are from the set of genetic loci. The method also includes determining, for each of the first reads having at least one potential locus, whether the first read aligns with a reference sequence of each of the potential genetic loci. The method also includes designating the first reads that do not align with a reference sequence as unaligned reads. The method also includes analyzing the unaligned reads to identify a genetic locus from the potential genetic loci that best fits with the unaligned read. The method also includes analyzing the unaligned reads to determine whether a potential allele dropout exists for the best-fit genetic locus.

In one aspect, the method may also include analyzing the unaligned reads to determine if the unaligned reads are one-on-target reads or pair-on-target reads. The pair-on-target reads may have first and second identifying sub-sequences that effectively match with first and second select sequences of a database. The one-on-target reads may have only the first identifying sub-sequence effectively matching the first select sequence of a database. Analyzing the unaligned reads to determine whether the potential allele dropout exists for the best-fit genetic locus may be based on a number of pair-on-target reads.

In an embodiment, a method is provided that includes receiving a read distribution for each genetic locus of a plurality of genetic loci. The read distribution includes a plurality of potential alleles, wherein each potential allele has an allele sequence and a read count. The read count represents a number of sample reads from sequencing data that were determined to include the potential allele. The method may also include identifying, for each genetic locus of the plurality of genetic loci, one of the potential alleles of the read distribution that has a maximum read count. The method may also include determining, for each genetic locus of the plurality of genetic loci, whether the maximum read count exceeds an interpretation threshold. If the maximum read exceeds the interpretation threshold, the method includes analyzing the potential allele(s) of the corresponding genetic locus to call a genotype for the genetic locus. If the maximum read is less than the interpretation threshold, the method includes generating an alert that the genetic locus has low coverage. The method also includes generating a genetic profile that has the genotypes for each of the genetic loci for which a genotype was called and the alert(s) for genetic loci that have low coverage.

In one aspect, analyzing the potential allele(s) of the corresponding genetic locus to call the genotype for the genetic locus may also include comparing a number of potential alleles for each genetic locus to a predetermined maximum number of allowable alleles for the genetic locus and generating an alert that the genetic locus has an excessive number of alleles if the number of potential alleles exceeds the predetermined maximum number of allowable alleles.

In another aspect, analyzing the potential allele(s) of the corresponding genetic locus to call the genotype for the genetic locus may also include generating an alert that the genetic locus is unbalanced if the genetic locus has a plurality of potential alleles that have insufficient proportions with respect to one another.

In another aspect, the method may also include determining, for each genetic locus of the plurality of genetic loci, whether the read counts of the potential alleles pass an analytical threshold. The analytical threshold may be easier to pass than the interpretation threshold.

In another aspect, the potential alleles having read counts that do not pass the interpretation threshold are designated as noise alleles, the method further comprising comparing a sum of the read counts of the noise alleles to a noise threshold and generating an alert that the genetic locus include excessive noise if the sum exceeds the noise threshold.

Optionally, the genetic loci include short tandem repeat (STR) loci and single nucleotide polymorphism (SNP) loci.

In an embodiment, a method is provided that includes: (a) receiving a read distribution for a genetic locus. The read distribution includes a plurality of potential alleles, wherein each potential allele has an allele sequence and a count score. The count score is based on a number of sample reads from sequencing data that were determined to include the potential allele. The method also includes: (b) determining whether the genetic locus has low coverage based on the count score of one more of the potential alleles. If the genetic locus has low coverage, the method includes generating a notice that the genetic locus has low coverage. If the genetic locus does not have low coverage, the method includes analyzing the count scores of the potential alleles to determine a genotype of the genetic locus. The method also includes: (d) generating a genetic profile that includes the genotype for the genetic locus or the alert that the genetic locus has low coverage.

In one aspect, determining whether the genetic locus has low coverage may include determining whether one or more of the count scores of the potential alleles passes an interpretation threshold. If at least one of the count scores passes the interpretation threshold, the method may also include analyzing the potential alleles of the corresponding genetic locus to call a genotype for the genetic locus. If none of the count scores passes the interpretation threshold, the method may include generating the notice that the genetic locus has low coverage.

In another aspect, determining whether the genetic locus has low coverage includes determining whether one or more of the count scores of the potential alleles passes an analytical threshold. If at least one of the count scores passes the analytical threshold, the method may also include analyzing the potential alleles of the corresponding genetic locus to call a genotype for the genetic locus. If none of the count scores passes the analytical threshold, the method may also include generating the notice that the genetic locus has low coverage.

In another aspect, determining whether the genetic locus has low coverage includes comparing a total number of aligned reads for the genetic locus to a read threshold. If the total number of aligned reads passes the read threshold, the method may include analyzing the potential alleles of the corresponding genetic locus to call a genotype for the genetic locus. If the total number of aligned reads does not pass the read threshold, the method may include generating the notice that the genetic locus has low coverage.

In another aspect, each of the count scores is a value that is equal to a read count for the corresponding potential allele.

In another aspect, each of the count scores is a function that is based on a read count and a total number of reads for the genetic locus.

In another aspect, each of the count scores is a function that is based on a read count and previously-obtained data of the genetic locus.

In another aspect, each of the count scores is a function that is based on read counts from other genetic loci of the sample.

In another aspect, each of the count scores is a function that is based on read counts of the genetic locus from other samples.

In another aspect, analyzing the potential alleles of the genetic locus to call the genotype for the genetic locus also includes comparing a number of potential alleles for the genetic locus to a predetermined maximum number of allowable alleles for the genetic locus and generating an alert that the genetic locus has an excessive number of alleles if the number of potential alleles exceeds the predetermined maximum number of allowable alleles.

In another aspect, analyzing the potential alleles of the genetic locus to call the genotype for the genetic locus may also include generating a notice that the genetic locus is unbalanced if the genetic locus has a plurality of potential alleles that have insufficient proportions with respect to one another.

In another aspect, the method may also include determining whether the count scores of the potential alleles pass an analytical threshold. The analytical threshold may be easier to pass than the interpretation threshold. Optionally, the potential alleles having count scores that do not pass the analytical threshold are designated as noise alleles. The method may also include comparing a noise score to a noise threshold and generating an alert that the genetic locus includes excessive noise if the noise score passes the noise threshold. The noise score may be based on the count scores of the noise alleles.

Optionally, the genetic locus is one of a short tandem repeat (STR) locus or a single nucleotide polymorphism (SNP) locus.

In another aspect, the method includes repeating (a)-(c) for a plurality of genetic loci, wherein generating the genetic profile includes calling a genotype for each of the genetic loci or providing a notice for each of the genetic loci having low coverage.

In an embodiment, a method is provided that includes receiving a read distribution for a genetic locus. The read distribution includes a plurality of potential alleles, wherein each potential allele has an allele sequence and a read count. The read count represents a number of sample reads from sequencing data that were assigned to the genetic locus. The method may also include determining a count score for each of the potential alleles. The count score may be based on the read count of the potential allele. The method may also include determining whether the count scores of the potential alleles pass an analytical threshold. If the count score of a corresponding potential allele does not pass the analytical threshold, the method includes discarding the corresponding potential allele. If the count score of a corresponding potential allele passes the analytical threshold, the method includes designating the potential allele as a designated allele of the genetic locus.

In one aspect, discarding the corresponding potential allele includes designating the potential allele as a noise allele. The method may also include determining whether the count scores of the noise alleles collectively pass a noise threshold. If the count scores collectively pass the noise threshold, the method may include generating an alert that the genetic locus has excessive noise.

In another aspect, each of the count scores is a value that is equal to the read count for the corresponding potential allele.

In another aspect, each of the count scores is a function that is based on the read count and a total number of reads for the genetic locus.

In another aspect, each of the count scores is a function that is based on the read count and previously-obtained data of the genetic locus.

In another aspect, the method may also include comparing a number of designated alleles to a predetermined maximum number of alllowable alleles for the genetic locus and generating an alert that the genetic locus has an excessive number of alleles if the number of designated alleles exceeds the predetermined maximum number of allowable alleles.

In another aspect, the method also includes generating an alert that the genetic locus is unbalanced if the genetic locus has a plurality of designated alleles that have insufficient proportions with respect to one another.

Optionally, the genetic loci include short tandem repeat (STR) loci and single nucleotide polymorphism (SNP) loci.

In an embodiment, a method is provided that includes receiving a read distribution for a genetic locus. The read distribution includes a plurality of potential alleles, wherein each potential allele has an allele sequence and a read count. The read count represents a number of sample reads from sequencing data that were assigned to the genetic locus. The method also includes determining whether the read counts exceed an analytical threshold. If the read count of a corresponding potential allele is less than the analytical threshold, the method includes designating the corresponding potential allele as a noise allele. If the read count of a corresponding potential allele passes the analytical threshold, the method includes designating the potential allele as an allele of the genetic locus. The method also includes determining whether a sum of the read counts of the noise alleles exceeds a noise threshold. If the sum exceeds the noise threshold, the method includes generating an alert that the genetic locus has excessive noise.

In one aspect, the method may also include comparing a number of designated alleles to a predetermined maximum number of allowable alleles for the genetic locus and generating an alert that the genetic locus has an excessive number of alleles if the number of designated alleles exceeds the predetermined maximum number of allowable alleles.

In another aspect, the method may also include generating an alert that the genetic locus is unbalanced if the genetic locus has a plurality of designated alleles that have insufficient proportions with respect to one another.

Optionally, the genetic loci include short tandem repeat (STR) loci and single nucleotide polymorphism (SNP) loci.

In an embodiment, a method is provided that includes receiving locus data for each genetic locus of a plurality of genetic loci. The locus data includes one or more designated alleles for the corresponding genetic locus. Each designated allele is based on read counts obtained from sequencing data. The method also includes determining, for each genetic locus of the plurality of genetic loci, whether a number of designated alleles for the corresponding genetic locus is greater than a predetermined maximum number of allowable alleles for the corresponding genetic locus. The method may include generating an allele-number alert if the number of designated alleles exceeds the predetermined maximum number of allowable alleles. The method also includes determining, for each genetic locus of the plurality of genetic loci, whether an allele proportion of the designated alleles is insufficient. The allele proportion may be based on read counts of the designated alleles. The method may also include generating an allele-proportion alert if the allele proportion is unbalanced. The method may also include determining that the sample includes a mixture of a plurality of sources based on a number of allele-number alert(s) and allele-proportion alerts(s) for the set of genetic loci.

In one aspect, determining that the sample includes a mixture of a plurality of sources includes determining that a total number of the alerts passes a mixture threshold. Optionally, the mixture threshold is based on a number of genetic loci in the set of genetic loci. Optionally, the mixture threshold is a predetermined value that is equal to a predetermined percentage of the genetic loci within the set.

In another aspect, generating an allele-number alert includes providing an allele-number score that is based on the number of designated alleles. Determining that the sample includes a mixture of a plurality of sources may include analyzing the allele-number score. Optionally, a likelihood of the sample including a mixture increases as the number of designated alleles beyond the maximum number of allowable alleles increases.

In another aspect, generating an allele-proportion alert includes providing an allele-proportion score that is based on the allele proportion. Determining that the sample includes a mixture of a plurality of sources includes analyzing the allele-proportion score. Optionally, a likelihood of the sample including a mixture increases as disproportion between the alleles increases.

Optionally, the genetic loci include short tandem repeat (STR) loci and single nucleotide polymorphism (SNP) loci.

In an embodiment, a method is provided that includes receiving locus data for a plurality of Y-loci. The locus data include designated alleles for the Y-loci. Each designated allele is based on read counts obtained from sequencing data. The method also includes comparing a number of designated alleles for each Y-locus to an expected number of alleles for the Y-loci. The method also includes generating a prediction that the sample is male or female based on results from the comparing operation. Optionally, the genetic loci include short tandem repeat (STR) loci and single nucleotide polymorphism (SNP) loci.

The present disclosure also envisions a system that includes a sample analyzer that is configured to carry out the method of one or more of the claims set forth herein.

Throughout this application various publications, patents and/or patent applications have been referenced.

As used herein, the terms "comprising," "including," and "having," and the like are intended to be open-ended, including not only the recited elements, but possibly encompassing additional elements.

As used in the description, the phrases "in an exemplary embodiment," "in some embodiments," "in particular embodiments," and the like means that the described embodiment(s) are examples of embodiments that may be formed or executed in accordance with the present application. The phrase is not intended to limit the inventive subject matter to that embodiment. More specifically, other embodiments of the inventive subject matter may not include the recited feature or structure described with a particular embodiment.

In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

## Claims

1. A method comprising:
receiving sequencing data including a plurality of sample reads that have corresponding sequences of nucleotides;
assigning the sample reads to designated loci based on the sequence of the nucleotides, wherein the sample reads that are assigned to a corresponding designated locus are assigned reads of the corresponding designated locus;
analyzing the assigned reads for each designated locus to identify corresponding regions-of-interest (ROIs) within the assigned reads, each of the ROIs having one or more series of repeat motifs in which each repeat motif of a corresponding series includes an identical set of the nucleotides;
sorting, for designated loci having multiple assigned reads, the assigned reads based on the sequences of the ROIs such that the ROIs with different sequences are assigned as different potential alleles, each potential allele having a sequence that is different from the sequences of other potential alleles within the designated locus;
analyzing, for designated loci having multiple potential alleles, the sequences of the potential alleles to determine whether a first allele of the potential alleles is suspected stutter product of a second allele of the potential alleles, the first allele being the suspected stutter product of the second allele if *k* repeat motifs within the corresponding sequences have been added or dropped between the first and second alleles, wherein *k* is a whole number, and wherein the total number of sample reads of the first allele is less than the total number of sample reads of the second allele; and
counting, for each designated locus having multiple potential alleles, a total number of the sample reads called for the first allele and for the second allele;
wherein, if the first allele is the suspected stutter product of the second allele:
(i) the first allele is the stutter product of the second allele if the total number of sample reads called for the first allele is within a predetermined percentile range of the total number of sample reads called for the second allele and no other mismatches exist between the sequences of the ROIs of the first and second alleles;
(ii) the first allele is from another contributor if the total number of sample reads called for the first allele is greater than the predetermined percentile range of the total number of sample reads called for the second allele; and
(iii) the first allele is designated as noise if the total number of sample reads called for the first allele is less than the predetermined percentile range of the total number of sample reads called for the second allele; and
wherein each of the steps in the method are performed using a suitably programmed computer.

2. The method of claim 1, wherein analyzing, for the designated loci having multiple potential alleles, the sequences of the potential alleles to determine whether the first allele is the suspected stutter product of the second allele includes comparing lengths of the ROIs of the first and second alleles to determine if the lengths of the ROIs of the first and second alleles differ by one repeat motif or multiple repeat motifs.

3. The method of claim 1 or claim 2, wherein analyzing, for the designated loci having multiple potential alleles, the sequences of the potential alleles to determine whether the first allele is the suspected stutter product of the second allele includes identifying the repeat motif(s) that have been added or dropped and determining whether the added or dropped repeat motif(s) is/are identical to an adjacent repeat motif in the corresponding sequences.

4. The method in accordance with any one of claims 1-3, wherein k is equal to 1 or 2.

5. The method in accordance with any one of claims 1-4, wherein the method further comprises generating a genotype profile, the genotype profile calling a genotype for at least a plurality of the designated loci, wherein the designated loci having suspected stutter product are indicated as having the suspected stutter product.

6. The method in accordance with any one of claims 1-5, wherein the method further comprises providing genotype calls for at least a plurality of the designated loci, wherein at least one of the genotype calls indicates that suspected stutter product exists for the designated locus of the at least one genotype call.

7. The method of any one of claims 1-6, wherein the predetermined percentile range is calculated using historical data or observations of the designated locus during the assay.

8. The method of any one of claims 1-7, wherein the predetermined percentile range is between about 5% and about 40% of the sample reads of the second allele.

9. The method of any one of claims 1-7, wherein the predetermined percentile range is between about 10% and about 30% of the sample reads of the second allele.

10. The method of any one of claims 1-7, wherein the predetermined percentile range is between about 10% and about 25% of the sample reads of the second allele.

11. The method in accordance with any one of claims 1-10, wherein the assigned reads include first and second conserved flanking regions having a corresponding repetitive segment located therebetween, wherein, for each assigned read, the method further comprises:
(a) providing a reference sequence comprising the first conserved flanking region and the second conserved flanking region;
(b) aligning a portion of the first flanking region of the reference sequence to the corresponding assigned read;
(c) aligning a portion of the second flanking region of the reference sequence to the corresponding assigned read; and
(d) determining the length and/or the sequence of the repetitive segment.

12. The method of claim 11, wherein the aligning a portion of the flanking region in one or both of steps (b) and (c) includes:
(i) determining a location of the corresponding conserved flanking region on the assigned read by using exact k-mer matching of a seeding region which overlaps or is adjacent to the repetitive segment; and
(ii) aligning the flanking region to the assigned read.

13. The method of claim 12, wherein the seeding region:
(a) comprises a high-complexity region of the conserved flanking region, wherein the high-complexity region incorporates each of the four nucleotides at a frequency of at least 10%, 15%, 20%, or 25% of all the nucleotides in the conserved flanking region; and/or
(b) does not comprise a low-complexity region of the corresponding conserved flanking region, wherein the low-complexity region comprises a sequence having more than 30%, 40%, 50%, 60%, 70% or more than 80% sequence identity to the repetitive segment.

14. The method in accordance with any one of claims 1-13 wherein the ROI includes a short tandem repeat (STR) selected from the group of loci consisting of CSF1PO, FGA, TH01, TPOX, VWA, D3S1358, D5S818, D7S820, D8S1179, D13S317, D16S539, D18S51, D21S11, PENTA D, and PENTA E.

## Patentansprüche

1. Ein Verfahren, das Folgendes beinhaltet:
Empfangen von Sequenzierungsdaten, die eine Vielzahl von Proben-Reads umfassen, die entsprechende Nukleotidsequenzen aufweisen;
Zuordnen der Proben-Reads zu designierten Loci, basierend auf der Sequenz der Nukleotide, wobei die Proben-Reads, die einem entsprechenden designierten Locus zugeordnet werden, zugeordnete Reads des entsprechenden designierten Locus sind;
Analysieren der zugeordneten Reads für jeden designierten Locus, um entsprechende Bereiche von Interesse (ROls, Regions-of-Interest) innerhalb der zugeordneten Reads zu identifizieren, wobei jeder der ROls eine oder mehrere Reihen von Wiederholungsmotiven aufweist, in denen jedes Wiederholungsmotiv einer entsprechenden Reihe einen identischen Satz der Nukleotide umfasst;
Sortieren, für designierte Loci, die mehrere zugeordnete Reads aufweisen, der zugeordneten Reads basierend auf den Sequenzen der ROls, sodass die ROls mit unterschiedlicher Sequenz als unterschiedliche potentielle Allele zugeordnet werden, wobei jedes potentielle Allel eine Sequenz aufweist, die sich von der Sequenz anderer potentieller Allele innerhalb des designierten Locus unterscheidet;
Analysieren der Sequenzen der potentiellen Allele für designierte Loci, die mehrere potentielle Allele aufweisen, um zu bestimmen, ob ein erstes Allel der potentiellen Allele das vermutete Stutter-Produkt eines zweiten Allels der potentiellen Allele ist, wobei das erste Allel das vermutete Stutter-Produkt des zweiten Allels ist, wenn k Wiederholungsmotive innerhalb der entsprechenden Sequenzen zwischen dem ersten und dem zweiten Allel hinzugefügt oder weggelassen wurden, wobei keine ganze Zahl ist und wobei die Gesamtzahl der Proben-Reads des ersten Allels geringer ist als die Gesamtzahl der Proben-Reads des zweiten Allels; und
Zählen einer Gesamtzahl der Proben-Reads, die für das erste Allel und für das zweite Allel aufgerufen wurden, für jeden designierten Locus mit mehreren potentiellen Allelen; wobei, wenn das erste Allel das vermutete Stutter-Produkt des zweiten Allels ist:
(i) das erste Allel das Stutter-Produkt des zweiten Allels ist, wenn die Gesamtzahl der Proben-Reads, die für das erste Allel aufgerufen werden, innerhalb eines vorbestimmten Perzentilbereichs der Gesamtzahl der Proben-Reads liegt, die für das zweite Allel aufgerufen werden, und keine anderen Nichtübereinstimmungen zwischen den Sequenzen der ROls des ersten und zweiten Allels existieren;
(ii) das erste Allel von einem anderen Kontributor stammt, wenn die Gesamtzahl der für das erste Allel aufgerufenen Proben-Reads größer ist als der vorbestimmte Perzentilbereich der Gesamtzahl der für das zweite Allel aufgerufenen Proben-Reads; und
(iii) das erste Allel als Rauschen designiert wird, wenn die Gesamtzahl der für das erste Allel aufgerufenen Proben-Reads geringer ist als der vorbestimmte Perzentilbereich der Gesamtzahl der für das zweite Allel aufgerufenen Proben-Reads; und
wobei jeder der Schritte in dem Verfahren unter Verwendung eines geeignet programmierten Computers durchgeführt wird.

2. Verfahren gemäß Anspruch 1, wobei das Analysieren der Sequenzen der potentiellen Allele für die designierten Loci, die mehrere potentielle Allele aufweisen, um zu bestimmen, ob das erste Allel das vermutete Stutter-Produkt des zweiten Allels ist, den Vergleich der Länge der ROls des ersten und zweiten Allels umfasst, um zu bestimmen, ob sich die Länge der ROls des ersten und zweiten Allels um ein Wiederholungsmotiv oder mehrere Wiederholungsmotive unterscheidet.

3. Verfahren gemäß Anspruch 1 oder Anspruch 2, wobei das Analysieren der Sequenzen der potentiellen Allele für die designierten Loci, die mehrere potentielle Allele aufweisen, um zu bestimmen, ob das erste Allel das vermutete Stutter-Produkt des zweiten Allels ist, das Identifizieren des Wiederholungsmotivs/der Wiederholungsmotive, das/die hinzugefügt oder weggelassen wurde(n), und das Bestimmen, ob das/die hinzugefügte(n) oder weggelassene(n) Wiederholungsmotiv(e) mit einem benachbarten Wiederholungsmotiv in den entsprechenden Sequenzen identisch ist/sind, umfasst.

4. Verfahren gemäß einem der Ansprüche 1-3, wobei k gleich 1 oder 2 ist.

5. Verfahren gemäß einem der Ansprüche 1-4, wobei das Verfahren ferner das Erzeugen eines Genotypprofils beinhaltet, wobei das Genotypprofil einen Genotyp für mindestens eine Vielzahl der designierten Loci aufruft, wobei die designierten Loci mit dem vermuteten Stutter-Produkt als solche mit dem vermuteten Stutter-Produkt angezeigt werden.

6. Verfahren gemäß einem der Ansprüche 1-5, wobei das Verfahren ferner das Bereitstellen von Genotypaufrufen für mindestens eine Vielzahl der designierten Loci beinhaltet, wobei mindestens einer der Genotypaufrufe anzeigt, dass das vermutete Stutter-Produkt für den designierten Locus des mindestens einen Genotypaufrufs existiert.

7. Verfahren gemäß einem der Ansprüche 1-6, wobei der vorbestimmte Perzentilbereich unter Verwendung historischer Daten oder Beobachtungen des designierten Locus während des Assays berechnet wird.

8. Verfahren gemäß einem der Ansprüche 1-7, wobei der vorbestimmte Perzentilbereich zwischen etwa 5 % und etwa 40 % der Proben-Reads des zweiten Allels liegt.

9. Verfahren gemäß einem der Ansprüche 1-7, wobei der vorbestimmte Perzentilbereich zwischen etwa 10 % und etwa 30 % der Proben-Reads des zweiten Allels liegt.

10. Verfahren gemäß einem der Ansprüche 1-7, wobei der vorbestimmte Perzentilbereich zwischen etwa 10 % und etwa 25 % der Proben-Reads des zweiten Allels liegt.

11. Verfahren gemäß einem der Ansprüche 1-10, wobei die zugeordneten Reads einen ersten und zweiten konservierten flankierenden Bereich mit einem dazwischen angeordneten entsprechenden sich wiederholenden Segment umfassen, wobei das Verfahren ferner für jeden zugeordneten Read Folgendes beinhaltet:
(a) Bereitstellen einer Referenzsequenz, die den ersten konservierten flankierenden Bereich und den zweiten konservierten flankierenden Bereich beinhaltet;
(b) Anlagern eines Abschnitts des ersten flankierenden Bereichs der Referenzsequenz an den entsprechenden zugeordneten Read;
(c) Anlagern eines Abschnitts des zweiten flankierenden Bereichs der Referenzsequenz an den entsprechenden zugeordneten Read; und
(d) Bestimmen der Länge und/oder der Sequenz des sich wiederholenden Segments.

12. Verfahren gemäß Anspruch 11, wobei das Anlagern eines Abschnitts des flankierenden Bereichs in einem oder beiden der Schritte (b) und (c) Folgendes umfasst:
(i) Bestimmen eines Ortes des entsprechenden konservierten flankierenden Bereichs auf dem zugeordneten Read durch Verwendung von exaktem k-mer-Matching eines Seeding-Bereichs, der das sich wiederholende Segment überlappt oder diesem benachbart ist; und
(ii) Anlagern des flankierenden Bereichs an den zugeordneten Read.

13. Verfahren gemäß Anspruch 12, wobei der Seeding-Bereich:
(a) einen hochkomplexen Bereich des konservierten flankierenden Bereichs beinhaltet, wobei der hochkomplexe Bereich jedes der vier Nukleotide mit einer Häufigkeit von mindestens 10 %, 15 %, 20 % oder 25 % aller Nukleotide in dem konservierten flankierenden Bereich enthält; und/oder
(b) keinen niederkomplexen Bereich des entsprechenden konservierten flankierenden Bereichs beinhaltet, wobei der niederkomplexe Bereich eine Sequenz mit mehr als 30 %, 40 %, 50 %, 60 %, 70 % oder mehr als 80 % Sequenzidentität zu dem sich wiederholenden Segment beinhaltet.

14. Verfahren gemäß einem der Ansprüche 1-13, wobei der ROI eine kurze Tandemwiederholung (STR, Short Tandem Repeat) umfasst, die aus der Gruppe von Loci ausgewählt ist, die aus CSF1PO, FGA, TH01, TPOX, VWA, D3S1358, D5S818, D7S820, D8S1179, D13S317, D16S539, D18S51, D21S11, PENTA D und PENTA E besteht.

## Revendications

1. Un procédé comprenant le fait :
de recevoir des données de séquençage incluant une pluralité de lectures d'échantillon qui ont des séquences correspondantes de nucléotides ;
d'assigner les lectures d'échantillon à des locus désignés sur la base de la séquence des nucléotides, où les lectures d'échantillon qui sont assignées à un locus désigné correspondant se voient assigner des lectures du locus désigné correspondant ;
d'analyser les lectures assignées pour chaque locus désigné afin d'identifier des régions d'intérêt (ROI) correspondantes au sein des lectures assignées, chacune des ROI ayant une ou plusieurs séries de motifs de répétition dans lesquelles chaque motif de répétition d'une série correspondante inclut un ensemble identique des nucléotides ;
de trier, pour des locus désignés ayant des lectures assignées multiples, les lectures assignées sur la base des séquences des ROI de telle sorte que les ROI avec des séquences différentes sont assignées comme des allèles potentiels différents, chaque allèle potentiel ayant une séquence qui est différente des séquences d'autres allèles potentiels au sein du locus désigné ;
d'analyser, pour des locus désignés ayant des allèles potentiels multiples, les séquences des allèles potentiels afin de déterminer si un premier allèle des allèles potentiels est un produit de bégaiement (« *stutter* ») suspecté d'un deuxième allèle des allèles potentiels, le premier allèle étant le produit de bégaiement suspecté du deuxième allèle si k motifs de répétition au sein des séquences correspondantes ont été ajoutés ou supprimés entre les premier et deuxième allèles, où *k* est un nombre entier, et où le nombre total de lectures d'échantillon du premier allèle est inférieur au nombre total de lectures d'échantillon du deuxième allèle ; et
de compter, pour chaque locus désigné ayant des allèles potentiels multiples, un nombre total des lectures d'échantillon appelées pour le premier allèle et pour le deuxième allèle ;
où, si le premier allèle est le produit de bégaiement suspecté du deuxième allèle :
(i) le premier allèle est le produit de bégaiement du deuxième allèle si le nombre total de lectures d'échantillon appelées pour le premier allèle se situe au sein d'un intervalle percentile prédéterminé du nombre total de lectures d'échantillon appelées pour le deuxième allèle et s'il n'existe pas d'autres mésappariements entre les séquences des ROI des premier et deuxième allèles ;
(ii) le premier allèle provient d'un autre contributeur si le nombre total de lectures d'échantillon appelées pour le premier allèle est supérieur à l'intervalle percentile prédéterminé du nombre total de lectures d'échantillon appelées pour le deuxième allèle ; et
(iii) le premier allèle est désigné comme du bruit si le nombre total de lectures d'échantillon appelées pour le premier allèle est inférieur à l'intervalle percentile prédéterminé du nombre total de lectures d'échantillon appelées pour le deuxième allèle ; et
où les étapes du procédé sont chacune réalisées en utilisant un ordinateur programmé de manière adéquate.

2. Le procédé de la revendication 1, où le fait d'analyser, pour les locus désignés ayant des allèles potentiels multiples, les séquences des allèles potentiels afin de déterminer si le premier allèle est le produit de bégaiement suspecté du deuxième allèle inclut le fait de comparer des longueurs des ROI des premier et deuxième allèles afin de déterminer si les longueurs des ROI des premier et deuxième allèles diffèrent par un motif de répétition ou des motifs de répétition multiples.

3. Le procédé de la revendication 1 ou de la revendication 2, où le fait d'analyser, pour les locus désignés ayant des allèles potentiels multiples, les séquences des allèles potentiels afin de déterminer si le premier allèle est le produit de bégaiement suspecté du deuxième allèle inclut le fait d'identifier le ou les motifs de répétition qui ont été ajoutés ou supprimés et le fait de déterminer si le ou les motifs de répétition ajoutés ou supprimés sont identiques à un motif de répétition adjacent dans les séquences correspondantes.

4. Le procédé conformément à n'importe laquelle des revendications 1 à 3, où *k* est égal à 1 ou 2.

5. Le procédé conformément à n'importe laquelle des revendications 1 à 4, où le procédé comprend en sus le fait de générer un profil de génotype, le profil de génotype appelant un génotype pour au moins une pluralité des locus désignés, où les locus désignés ayant un produit de bégaiement suspecté sont indiqués comme ayant le produit de bégaiement suspecté.

6. Le procédé conformément à n'importe laquelle des revendications 1 à 5, où le procédé comprend en sus le fait de fournir des appels de génotype pour au moins une pluralité des locus désignés, où au moins un des appels de génotype indique qu'il existe un produit de bégaiement suspecté pour le locus désigné de l'au moins un appel de génotype.

7. Le procédé de n'importe laquelle des revendications 1 à 6, où l'intervalle percentile prédéterminé est calculé en utilisant des données historiques ou des observations du locus désigné durant l'essai.

8. Le procédé de n'importe laquelle des revendications 1 à 7, où l'intervalle percentile prédéterminé se situe entre environ 5 % et environ 40 % des lectures d'échantillon du deuxième allèle.

9. Le procédé de n'importe laquelle des revendications 1 à 7, où l'intervalle percentile prédéterminé se situe entre environ 10 % et environ 30 % des lectures d'échantillon du deuxième allèle.

10. Le procédé de n'importe laquelle des revendications 1 à 7, où l'intervalle percentile prédéterminé se situe entre environ 10 % et environ 25 % des lectures d'échantillon du deuxième allèle.

11. Le procédé conformément à n'importe laquelle des revendications 1 à 10, où les lectures assignées incluent des première et deuxième régions flanquantes conservées ayant un segment répétitif correspondant placé entre, où, pour chaque lecture assignée, le procédé comprend en sus le fait :
(a) de fournir une séquence de référence comprenant la première région flanquante conservée et la deuxième région flanquante conservée ;
(b) d'aligner une portion de la première région flanquante de la séquence de référence par rapport à la lecture assignée correspondante ;
(c) d'aligner une portion de la deuxième région flanquante de la séquence de référence par rapport à la lecture assignée correspondante ; et
(d) de déterminer la longueur et/ou la séquence du segment répétitif.

12. Le procédé de la revendication 11, où le fait d'aligner une portion de la région flanquante dans l'une ou l'autre ou l'une et l'autre des étapes (b) et (c) inclut le fait :
(i) de déterminer un emplacement de la région flanquante conservée correspondante sur la lecture assignée en utilisant un appariement de k-mers exact d'une région de seeding qui chevauche ou qui est adjacente au segment répétitif ; et
(ii) d'aligner la région flanquante par rapport à la lecture assignée.

13. Le procédé de la revendication 12, où la région de seeding :
(a) comprend une région d'importante complexité de la région flanquante conservée, où la région d'importante complexité incorpore chacun des quatre nucléotides à une fréquence d'au moins 10 %, 15 %, 20 %, ou 25 % de tous les nucléotides dans la région flanquante conservée ; et/ou
(b) ne comprend pas une région de faible complexité de la région flanquante conservée correspondante, où la région de faible complexité comprend une séquence ayant une identité de séquence de plus de 30 %, 40 %, 50 %, 60 %, 70 % ou de plus de 80 % par rapport au segment répétitif.

14. Le procédé conformément à n'importe laquelle des revendications 1 à 13 où la ROI inclut une répétition en tandem courte (STR) sélectionnée dans le groupe de locus constitué de CSF1PO, FGA, TH01, TPOX, VWA, D3S1358, D5S818, D7S820, D8S1179, D13S317, D16S539, D18S51, D21S11, PENTA D, et PENTA E.
